# EUROPEAN PATENT SPECIFICATION

(11) **EP 1 976 997 B1**
(45) Date of publication and mention of the grant of the patent: **08.04.2009**
(21) Application number: 07712041.8
(22) Date of filing: 16.01.2007
(51) Int. Cl.: C12P 33/08, C07J 7/00

(54) **PROCESS FOR THE PREPARATION OF 11BETA-HYDR0XY-9BETA,10ALPHA-STEROIDS EMPLOYING AMYCOLATOPSIS MEDITERRANEI CELLS**
VERFAHREN ZUR HERSTELLUNG VON 11BETA-HYDR0XY-9BETA,10ALPHA-STEROIDEN UNTER VERWENDUNG VON AMYCOLATOPSIS MEDITERRANEI-ZELLEN
PROCÉDÉ DE PRÉPARATION DE 11BETA-HYDROXY-9BETA,10ALPHA-STÉROÏDES EN UTILISANT DES CELLULES DE AMYCOLATOPSIS MEDITERRANEI

(30) Priority: 18.01.2006 EP 06100503
(43) Date of publication of application: 08.10.2008
(73) Proprietor: Solvay Pharmaceuticals GmbH, 30173 Hannover (DE)
(72) Inventor: MESSINGER, Josef, 31319 Sehnde (DE); THOLE, Heinrich-Hubert, 30655 Hannover (DE); RASCHE, Heinz-Helmer, 31303 Burgdorf (DE); SCHMIDT, Michael, 32657 Lemgo (DE); HAKALA, Juha, FI-20540 Turku (FI)
(74) Representative: Gosmann, Martin
(86) International application number: PCT/EP2007/050412
(87) International publication number: WO 2007/082891

(56) References cited:
- GB-A- 1 111 320

## Description

### FIELD OF INVENTION

The present invention relates to the use of bacterial strains of the species *Amycolatopsis mediterranei* for the microbial transformation of 9β,10α-steroids (retrosteroids) to their corresponding 11β-hydroxyl analogues, as well as to a specific strain of that species. In addition, the present invention relates to the process of transforming 9β,10α-steroids (retrosteroids) to their corresponding 11β-hydroxyl derivatives using bacterial strains of the species *Amycolatopsis mediterranei,* and to the subsequent isolation of the 11β-hydroxyl derivatives from the bacterial culture medium. The resultant 11β-hydroxylated products are useful intermediates for the preparation of novel steroidal compounds with 9β,10α-conformation carrying different kind of substituents in the 11β-position.

### BACKGROUND OF THE INVENTION

### Retrosteroids

Retrosteroids, i.e. steroids with 9β, 10α-conformation, are well known in the state of the art. The commercially available compound Dydrogesterone ((9β,10α)-Pregna-4,6-diene-3,20-dione) of the following formula (I-1) 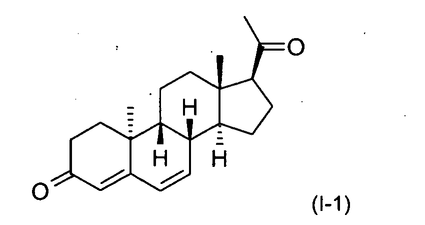 is an orally active progestative hormone and is generally used to correct deficiencies of progesterone in the body. The synthesis of Dydrogesterone by irradiation and photochemical reaction is for example described within European patents EP0152138B1 (US 4,601,855) and EP0558119B1 (US 5,304,291).

Further known retrosteroids are for example 1,2-methylene-3-keto-Δ^{4,6}-bisdehydro-6-halo-9β,10α-steroids as disclosed within US 3,937,700 and 3-keto-Δ^{4,6}-bisdehydro-6-halo-9β,10α-steroids as described within BE 652,597 and US 3,304,314. Furthermore, the patent US 3,555,053 describes a process for the preparation of 6-halo- or 6-alkyl-9β,10α-steroids. Some 6,7-dehydro-9β,10α steroids are described by Westerhof & Hartog [1965]. The synthesis of further retrosteroids is disclosed within Hartog et al. [1972] for some 16-methylene-17α-acetoxy-9β,10α-pregna-4,6-diene-3,20-dione derivatives and within Halkes et al [1972] for 1,2β-methylene-17α-acetoxy-9β,10α-pregnanes. In addition, 18-alkyl-9β,10α-pregnane derivatives are disclosed by Van Moorselaar & Halkes [1969]. However, the retrosteroidal compounds known so far were all developed for having progestational activity, i.e. being progesterone receptor agonists.

Further retrosteroids showing hormonal activity and carrying hydroxy or esterified hydroxy substituents in the C11 position where already described within GB 1,111,320. Compounds or intermediates especially described are
11β-Hydroxy-9β,10α-pregna-4,6-diene-3,20-dione (CAS No. 22413-62-3),
11 β-Hydroxy-9β,10α-pregna-4-ene-3,20-dione (CAS No. 10007-43-9), and
11β-17α-Dihydroxy-9β,10α-pregna-4-ene-3,20-dione (CAS No. 4076-89-5),
as well as the 11β-acetoxy derivatives thereof which were obtained from the corresponding 11β-hydroxy compounds by chemical modification.

Since compounds which are agonists, partial agonists (i.e., partial activators and/or tissue-specific activators) and/or antagonists for progesterone receptors, preferably showing a balanced agonistic / antagonistic profile, are regarded to be of significant value for the improvement of women's health, there still remains a need for the development of novel compounds which therapeutically modulate the progesterone receptor with an improved agonistic and/or antagonistic mode, which show with higher receptor-selectivity for the progesterone over other steroid hormone receptors than currently known compounds, and which provide a good tissue-selectivity (e.g. selectivity for uterine tissue over breast tissue). Retrosteroidal compounds carrying different kind of substituents in the C11β-position might fulfill this aim. However, despite the compounds disclosed in GB 1,111,320 no retrosteroidal derivatives carrying substituents in the C11β-position have been disclosed so far.

Accordingly, a major aspect of the present invention is to provide key intermediate compounds that are useful for the preparation of novel steroidal compounds with 9β,10α-conformation carrying different kind of substituents in the C11β-position as well as a process to obtain the desired intermediates in high yield and quantity. These key intermediates preferably possess a hydroxy group in the C11β-position of the steroidal core and comprise 11β-Hydroxy-dydrogesterone, 11β-Hydroxy-9β, 10α-progesterone and derivatives thereof.

### Microbial Transformation - Hydroxylation of the C11 position of the steroidal core

The microbial transformation of steroidal compounds, including the hydroxylation of the C11-position of the steroidal core, is a process well known in the state of the art. Typically, fungal strains of the species *Aspergillus* or *Rhizopus* are used for 11α-hydroxylation of steroids with 9α,10β-conformation (as disclosed e.g. in European patent application EP0028309, and US patent No. 6,046,023). The 11β-hydroxylation of steroids with 9α,10β-conformation is achievable by using funghi such as of the genus Curvularia (US patent No. 4,353,985), or more specifically *Curvularia lunata* (US patent No. 4,588,683), or *Cochliobolus lunatus* [Zakelj-Mavric et al., 1990].

### Hydroxylation of Retrosteroids

The publication by van der Sijde et al [1966] deals with the C11-hydroxylation of retrosteroids, for example of 9β,10α-progesterone, with the fungal strain Aspergillus ochraceus NRRL405. However, the hydroxylation of the retrosteroidal core occurred in the 11 α-position.

Another publication by Saucy et al [1966] also discloses the microbial hydroxylation of retrosteroids in the C11 position while using *Aspergillus ochraceus* for 11α-hydroxylation and an unrevealed microorganism for 11β-hydroxylation.

The patent specification GB 1,111,320 disclosed the microbial hydroxylation of some specific retrosteroids in the C11 position. In particular, the description revealed a process for the manufacture of some 11β-hydroxy-retrosteroids comprising fermenting a corresponding 11-unsubstituted retrosteroid with a microorganism of the taxonomic subgroup Funghi imperfecti, Ascomycetes, Phytomycetes, Basidiomycetes or Actinomycetales. Specific strains of the aforementioned taxonomic subgroups especially suitable for carrying out the hydroxylation process include *Gliocladium catenulatum, Gliocladium roseum, Helicostylum piriforme, Penicillium canescens, Mucor griseocyanus, Mucor corymbifer, Choanephora circinans, Nocardia lurida (= Amycolatopsis orientalis subsp. lurida), Streptomyces rimosus and Streptomyces fradiae.* The provided examples show the hydroxylation of 9β,10α-pregna-4,6-diene-3,20-dione (Dydrogesterone), 9β,10α-pregna-4-ene-3,20-dione (9β,10α-Progesterone) or 17α-Hydroxy-9β,10α-pregna-4-ene-3,20-dione, i.e. retrosteroids corresponding or most similar to Dydrogesterone, using the following strains: *Nocardia lurida, Penicillium canescens, Gliocladium catenulatum, Helicostylum piriforme, Choanephora circinans, Streptomyces fradiae, Mucor griseocyanus, and Streptomyces rimosus.* A major drawback of the disclosed fermentation process was the relatively low yield of the desired fermentation product, which typically lay between 2 to 10%, in one example up to 30% of the corresponding educt.

### Amycolatopsis mediterranei

The bacterial species *Amycolatopsis mediterranei* was formerly also known under the names *Streptomyces mediterranei* and *Nocardia mediterranei* [Margalith and Beretta, 1960, and Lechevalier et al. 1986]. *Amycolatopsis mediterranei* belongs to the class of Actinobacteria, in particular to the taxonomic subgroup Actinomycetales, and to the genus Amycolatopsis. Several strains are known from this species and available from public culture collections like the "Deutsche Sammlung von Mikroorganismen und Zellkulturen", the DSMZ (Address: Mascheroder Weg 1b, D-38124 Braunschweig, Germany) such as DSM 43304 (also deposited in other culture collections under ATCC 13685, CBS 121.63, CBS 716.72, DSM 40501, IFO 13415, IMET 7651, ISP 5501, JCM 4789, KCC S-0789, LBG A 3136, NBRC 13142, NBRC 13415, NCIB 9613, NRRL B-3240, RIA 1376, or VKM Ac-798), DSM 40773, and DSM 46096 (also deposited in other culture collections under ATCC 21411, IMET 7669).

*Amycolatopsis mediterranei* strains are well known for the microbiological production of the antibiotically active compound Rifamycin B; however this species was so far not described for the hydroxylation of steroidal compounds and in particular not for the C11 β-hydroxylation of retrosteroids.

### Process for the purification of steroids from microbial cultures

The product of a fermentation process is typically isolated from the culture medium by a multi-step process of filtration (removal of the mycelium), extraction, optionally chromatographic purification, crystallization and subsequent recrystallization in order to obtain a pure compound. For example, the microbial hydroxylation products as disclosed in the patent specification GB 1,111,320 are obtained from the fermentation batch by a quite elaborated procedure including several liquid-liquid extraction steps and subsequent column chromatography or recrystallization for further purification, thereby using toxic and/or non-healthy organic solvents such as benzene or carbon tetrachloride.

Accordingly, there still remains a need for an optimized process for the microbial 11β-hydroxylation of a retrosteroidal compound, and in particular for identifying a bacterial species and corresponding strains which are able to carry out this hydroxylation process with high efficacy and high yield.

### SUMMARY OF THE INVENTION

The aim of the present invention was to develop a new microbial transformation method for the easy and quantitative production of retrosteroidal compounds carrying a C11β-hydroxyl group, which compounds are useful as key intermediates for the synthesis for novel progesterone receptor modulator compounds based on the retrosteroidal core of the known progesterone agonist Dydrogesterone. Therefore, it is an object of the present invention to identify a microbial species which is capable of the 11β-hydroxylation of retrosteroids identical and similar to Dydrogesterone with high efficiency and high yield.

Surprisingly it was found that by using a bacterial microorganism of the species *Amycolatopsis mediterranei* a 9β,10α-steroidal (retrosteroidal) compound of general Formula (I) 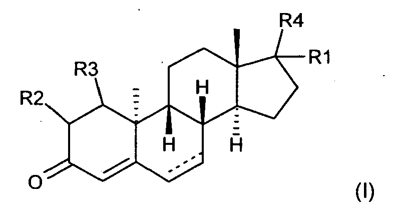 wherein
R1 and R4 together form an oxygen, or
R4 is a β-acetyl group and R1 is selected from the group consisting of hydrogen, -OH, -0-(C₁-C₄)alkyl, and -O-CO-(C₁-C₄)alkyl, and
R2 and R3 are both hydrogen or together form a methylene group,
can be transformed into its corresponding 11β-hydroxyl analogue.

In one embodiment, the 9β,10α-steroidal (retrosteroidal) compound used in the aforementioned transformation is represented by a compound of general Formula (II) 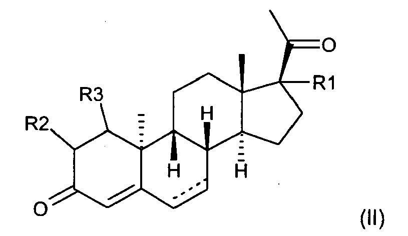 wherein
R1 is selected from the group consisting of hydrogen, -OH, -O-(C₁-C₄)alkyl, and -O-CO-(C₁-C₄)alkyl; and
R2 and R3 are both hydrogen or together form a methylen group.

A further embodiment relates to the use of a bacterial microorganism of the species *Amycolatopsis mediterranei* for the aforementioned transformation, which bacterial microorganism is a strain selected from the group consisting of *Amycolatopsis mediterranei* LS30, DSM 43304 (corresponding to ATCC 13685, CBS 121.63, CBS 716.72, DSM 40501, IFO 13415, IMET 7651, ISP 5501, JCM 4789, KCC S-0789, LBG A 3136, NBRC 13142, NBRC 13415, NCIB 9613, NRRL B-3240, RIA 1376 or VKM Ac-798), DSM 40773, and DSM 46096 (corresponding to ATCC 21411, IMET 7669). The preferably used microorganism is bacterial strain *Amycolatopsis mediterranei* LS30 as deposited under DSM 17416 at the "Deutsche Sammlung von Mikroorganismen und Zellkulturen", the DSMZ (Address: Mascheroder Weg 1b, D-38124 Braunschweig, Germany).

One particular microorganism used for said microbial transformation is the bacterial strain *Amycolatopsis mediterranei* LS30 which was newly identified and not described before in the literature. The bacterial strain LS30 was characterized as belonging to the species *Amycolatopsis mediterranei* based on macroscopic and microscopic appearance (colony morphology), based on chemotaxonomic classification (fatty acid pattern) and based on comparative 16S rRNA sequencing. Accordingly, the present invention also relates to the bacterial strain *Amycolatopsis mediterranei* LS30 as deposited under DSM 17416 at the "Deutsche Sammlung von Mikroorganismen und Zellkulturen", the DSMZ (Address: Mascheroder Weg 1b, D-38124 Braunschweig, Germany).

When transforming a retrosteroidal compound of general Formula (I), an 11β-hydroxyl analogue thereof will be obtained as transformation product that is represented by the following general Formula (IV) 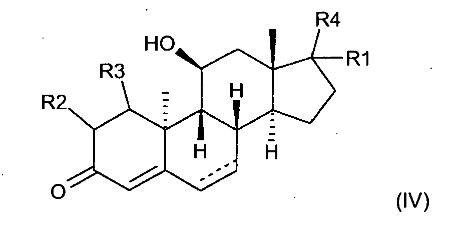 wherein
R1 and R4 together form an oxygen, OR R4 is a β-acetyl group and R1 is selected from the group consisting of hydrogen, -OH, -O-(C₁-C₄)alkyl, and -O-CO-(C₁-C₄)alkyl, and
R2 and R3 are both hydrogen or together form a methylen group.

When transforming a retrosteroidal compound of general Formula (II), an 11β-hydroxyl analogue thereof will be obtained as transformation product that is represented by the following general Formula (V) 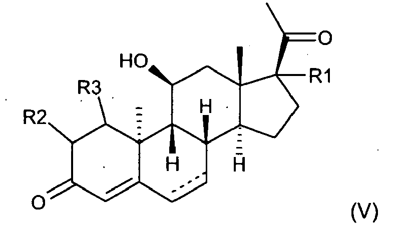 wherein
R1 is selected from the group consisting of hydrogen, -OH, -O-(C₁-C₄)alkyl, and -O-CO-(C₁-C₄)alkyl, and
R2 and R3 are both hydrogen or together form a methylen group.

Said compounds of general formulas (IV) and (V) are the desired key intermediate compounds that are useful for the preparation of novel steroidal compounds with 9β,10α-conformation carrying different kind of substituents in the C11β position.

Some of the particular compounds falling under the scope of general formula (V) are already known, such as 11β-Hydroxy-9β,10α-pregna-4,6-diene-3,20-dione (CAS No. 22413-62-3), 11β-Hydroxy-9β,10α-pregna-4-ene-3,20-dione (CAS No. 10007-43-9), and 11β-17α-Dihydroxy-9β,10α-pregna-4-ene-3,20-dione (CAS No. 4076-89-5); however, the remaining compounds of general formula (V) are novel and do also form part of the present invention.

Accordingly, it is a further object of the present invention to provide 11β-hydroxy-retrosteroidal compounds of general formula (V) 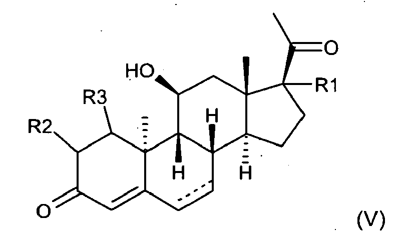 wherein
a) R1 is selected from the group consisting of hydrogen, -OH, -O-(C₁-C₄)alkyl, and -O-CO-(C₁-C₄)alkyl, and R2 and R3 together form a methylen group, OR
b) R1 is selected from the group consisting of -O-(C₁-C₄)alkyl and -O-CO-(C₁-C₄)alkyl, and R2 and R3 both represent hydrogen; OR
c) R1 is -OH, R2 and R3 both represent hydrogen, and the compound is a 4,6-diene.

In one embodiment, the 11β-hydroxy-retrosteroidal intermediate compound is selected from the group consisting of
11β-Hydroxy-1,2-methylene-9β, 10α-pregna-4,6-diene-3,20-dione,
17α-Ethoxy-11β-hydroxy-9β,10α-pregna-4,6-diene-3,20-dione,
17α-Ethoxy-11β-hydroxy-1,2-methylene-9β,10α-pregna-4,6-diene-3,20-dione,
11β-17α-Dihydroxy-9β,10α-pregna-4,6-diene-3,20-dione,
11β-17α-Dihydroxy-1,2-methylene-9β,10α-pregna-4,6-diene-3,20-dione,
11β-Hydroxy-1,2-methylene-9β, 10α-pregna-4-ene-3,20-dione,
17α-Ethoxy-11β-hydroxy-9β,10α-pregna-4-ene-3,20-dione,
17α-Ethoxy-11β-hydroxy-1,2-methylene-9β,10α-pregna-4-ene-3,20-dione, and
11β-17α-Dihydroxy-1,2-methylene-9β,10α-pregna-4-ene-3,20-dione.

Since the aim of the present invention was the development of a new and improved process for the delivery of said intermediate compounds, a further aspect of the present invention relates to a process for the microbial *in vitro* transformation of a retrosteroidal compound of general Formula (1) 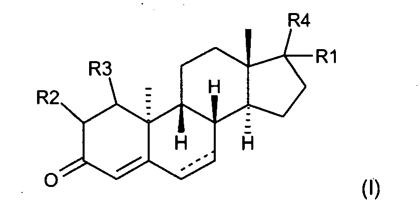 wherein
R1 and R4 together form an oxygen, or
R4 is a β-acetyl group and R1 is selected from the group consisting of hydrogen, -OH, -0-(C₁-C₄)alkyl, and -O-CO-(C₁-C₄)alkyl, and
R2 and R3 are both hydrogen or together form a methylen group; into its corresponding 11β-hydroxyl analogue, which process comprises contacting a compound of Formula (I) in a (suitable) fermentation medium with a bacterial member of the species *Amycolatopsis mediterranei* capable of performing the transformation of a compound of Formula (I) into its corresponding 11β-hydroxyl analogue.

In one embodiment, the present invention relates to a process for the microbial *in vitro* transformation of a retrosteroidal compound of general Formula (II) 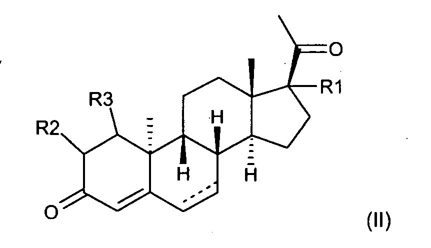 wherein
R1 is selected from the group consisting of hydrogen, -OH, -O-(C₁-C₄)alkyl, and -O-CO-(C₁-C₄)alkyl; and
R2 and R3 are both hydrogen or together form a methylen group, into its corresponding 11β-hydroxyl analogue, which process comprises contacting a compound of Formula (II) in a (suitable) fermentation medium with a bacterial member of the species *Amycolatopsis mediterranei* capable of performing the transformation of a compound of Formula (II) into its corresponding 11β-hydroxyl analogue.

A further embodiment of the present invention relates to a process for the microbial *in vitro* transformation of a 9β,10α-steroidal compound of general Formula (III) 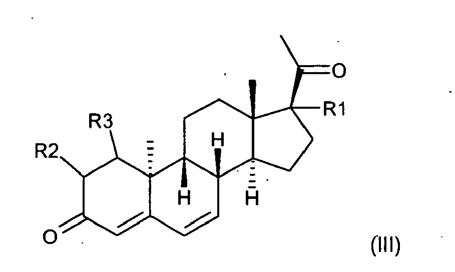 wherein
R1 is hydrogen or -O-(C₁-C₄)alkyl; and
R2 and R3 are both hydrogen or together form a methylen group, into its corresponding 11β-hydroxyl analogue, which process comprises contacting a compound of Formula (III) in a (suitable) fermentation medium with a bacterial member of the species *Amycolatopsis mediterranei* capable of performing the transformation of a compound of Formula (III) into its corresponding 11β-hydroxyl analogue.

Furthermore, the present inventions deals with an optimized method for isolating the desired 11β-hydroxy-retrosteroidal compounds of general formulas (IV) and/or (V) as defined above from the fermentation broth. Accordingly, the present invention also relates to a process for the isolation of said 11β-hydroxyl analogue from the fermentation medium (= bacterial culture medium) after transformation of the retrosteroidal compound of general Formulas (I), (II) or (III) as defined above with a member of the species *Amycolatopsis mediterranei,* whereby the 11β-hydroxyl analogue is isolated from the fermentation medium by a process comprising the steps of
a) obtaining the supernatant from the fermentation medium optionally freed of any bacterial cells, bacterial debris, mucilaginous substances and solids,
b) contacting a supernatant material selected from the group consisting of the supernatant obtained in step a), a concentrate formed by reducing the volume of said supernatant, and a retentate obtained by membrane filtration of said supernatant, with an amount of a non-ionic semi-polar polymeric adsorber resin sufficient for the adsorption of the 11β-hydroxyl analogue contained in the supernatant material, whereby a non-ionic semi-polar polymeric adsorber resin charged with the 11β-hydroxyl analogue is obtained, and thereafter separating the charged adsorber resin from the rest of the supernatant material;
c) washing the charged adsorber resin with an alkaline aqueous washing liquid having a pH value of at least 12.0, preferably of from 12.5 to 14;
d) optionally performing an intermediate washing step, in which the charged adsorber resin is washed with water; and
e) contacting the washed adsorber resin with an amount of an elution liquid sufficient for the desorption of the 11β-hydroxyl analogue adsorbed thereon, said elution liquid comprising at least one water-miscible organic solvent selected from the group consisting of water-miscible ethers, lower alkanols, and lower aliphatic ketones or a mixture of water which has optionally been rendered alkaline and at least one water-miscible organic solvent selected from the group consisting of water-miscible ethers, lower alkanols and lower aliphatic ketones, and
f) separating an eluate containing the 11β-hydroxyl analogue off from the adsorber resin, and optionally concentrating the eluat by volume reduction.

### DETAILED DESCRIPTION OF THE INVENTION

### Definitions:

As used herein, the following terms are defined with the following meanings, unless explicitly stated otherwise.

As used herein, the terms "comprising" and "including" are used herein in their open, non-limiting sense.

The word "compound" shall here be understood to cover any and all isomers (e. g., enantiomers, stereoisomers, diastereomers, rotomers, tautomers) or any mixture of isomers, prodrugs, and any pharmaceutically acceptable salt of said compound, unless stated otherwise.

Where the plural form is used for compounds, salts, and the like, this is taken to mean also a single compound, salt, or the like.

The compounds of the invention may contain at least one asymmetric centre on the molecule, e.g. a chiral carbon atom, depending upon the nature of the various substituents. In case of such an asymmetric centre, the compounds could thus be present in two optically active stereoisomeric forms or as a racemate. The present invention should include both the racemic mixtures and the isomerically pure compounds, unless specifically indicated in writing or within the structural formulas displayed, as for example for the 9β,10α-conformation or for the C11β conformation or for the C17β-acetyl group. The compounds of the present invention may contain further asymmetric centres on the molecule, depending upon the nature of the various substituents. In certain instances, asymmetry may also be present due to restricted rotation about the central bond adjoining the two aromatic rings of the specified compounds. It is intended that all isomers (including enantiomers and diastereomers), either by nature of asymmetric centres or by restricted rotation as described above, as separated, pure or partially purified isomers or racemic mixtures thereof, be included within the ambit of the instant invention.

Any asymmetric carbon atoms may be present in the (R)-, (S)- or (R,S)-configuration, preferably in the (R)- or (S)-configuration, whichever is most active. Substituents at a double bond or a ring may be present in cis- (.=Z-) or trans (=E-) form.

The term "retrosteroid" refers to a steroidal compound with 9β,10α conformation.

The term "substituted" means that the specified group or moiety bears one or more substituents. Where any group may carry multiple substituents and a variety of possible substituents is provided, the substituents are independently selected and need not be the same. The term "unsubstituted" means that the specified group bears no substituents. The term "optionally substituted" means that the specified group is unsubstituted or substituted by one or more substituents.

The term "hydroxyl" or "hydroxy" refers to the group -OH

The term "alkyl" stands for a hydrocarbon radical which may be linear, cyclic or branched, with single or multiple branching, whereby the alkyl group in general comprises 1 to 12 carbon atoms. In one embodiment, the term "alkyl" stands for a linear or branched (with single or multiple branching) alkyl chain of 1 to 4 carbon atoms, exemplified by the term (C₁-C₄)alkyl. The term (C₁-C₄)alkyl is further exemplified by such groups as methyl; ethyl; n-propyl; isopropyl; n-butyl; sec-butyl; isobutyl; and tert-butyl. The alkyl or (C₁-C₄)alkyl group may be partially unsaturated, forming such groups as, for example, vinyl, 1-propenyl, 2-propenyl (allyl), and butenyl. The term "alkyl" further comprises cycloalkyl groups, preferably cyclo(C₃-C₄)alkyl which refers to cyclopropyl or cyclobutyl, and isomeric forms thereof such as methylcyclopropyl. The cycloalkyl group may also be partly unsaturated. Furthermore, the term (C₁-C₄)alkyl also comprises a cyclopropylmethyl group.

The term "methylene" refers to -CH₂-.

The term "acetyl" refers to -CO-CH₃.

### Compound Numbering (Nomenclature)

Furthermore, in an effort to maintain consistency in the naming of compounds of similar structure but differing substituents, the compounds described herein are named according to the following general guidelines. The numbering system for the location of substituents on such compounds is also provided.

The C-atoms of the steroidal core of the pregnane derivate are numbered according to the following general scheme:

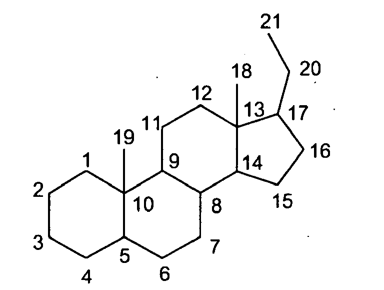

Dydrogesterone-9β,10α-Pregna-4,6-diene-3,20-dione - has the following formula (I-1): .

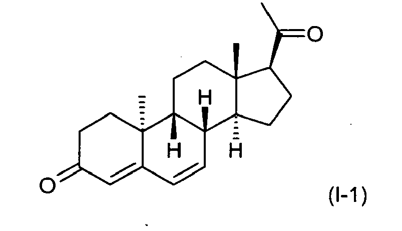

Retroprogesterone - 9β,10α-Pregna-4-ene-3;20-dione - has the following formula (I-3):

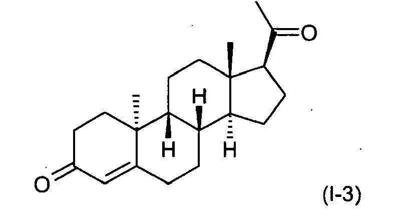

General structural formulas are typically designated with a number in Roman format I, II, III etc. Intermediates are indicated with the same numbers in Roman format as of the corresponding general formulas and a further letter or number, e.g. I-2 for a particular derivative falling under the scope of general formula I. The compounds of the invention are designated No.1, No.2 etc.

### Abbreviations and Acronyms

- BV: bed volume
- d: day(s)
- DCM: dichloromethane
- DDQ: 2,3-dichloro-5,6-dicyanonezoquinone
- DM: dry matter
- DMSO: dimethyl sulphoxide
- DSMZ: Deutsche Sammlung von Mikroorganismen und Zellkulturen
- h: hour(s)
- HPLC: high performance liquid chromatography
- LAH: lithium aluminium hydride
- min: minutes
- NMMO: N-methylmorpholine-N-oxide
- rpm: revolutions per minute

### Educts of general formula (I)

A 9β,10α-steroidal (retrosteroidal) compound of general Formula (I) 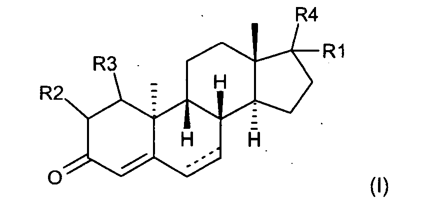 wherein
R1 and R4 together form an oxygen, or
R4 is an β-acetyl group and R1 is selected from the group consisting of hydrogen, -OH, -0-(C₁-C₄)alkyl, and -O-CO-(C₁-C₄)alkyl, and
R2 and R3 are both hydrogen or together form a methylen group,
and used as starting material in the processes of the present invention, may be prepared from known retrosteroids by use of known chemical reactions and procedures. Nevertheless, the following general preparative methods are presented to aid the reader in synthesizing the educts used in the present invention. All variable groups of these methods are as described in the generic description if they are not specifically defined below. '

It is recognized that some educts of the invention with each claimed optional functional group may not be prepared by each of the below-listed methods. Within the scope of each method, optional substituents may appear on reagents or intermediates which may act as protecting or otherwise non-participating groups. Utilizing methods well known to those skilled in the art, these groups are introduced and/or removed during the course of the synthetic schemes which provide the compounds of the present invention.

The sequence of steps for the general schemes to synthesize the compounds of the present invention is shown below. In each of the Schemes the R groups (e. g., R1, R2, etc.) correspond to the specific substitution patterns noted in the Description and the Examples.

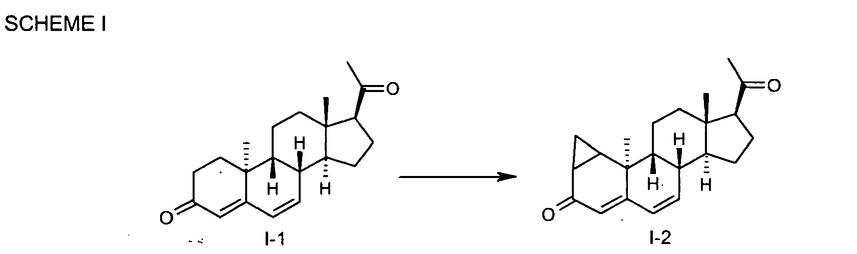

Scheme I shows the optional reaction that commercially available Dydrogesterone (9β,10α-pregna-4,6-diene-3,20-dione) of formula (I-1) is substituted in the 1,2 position with a methylene group. The introduction of the 1,2-methylene group might be performed according to the well known procedures as described by Halkes et al [1972] and within US patent No. 3,937,700 for 17α-Hydroxy-9β,10α-pregna-4,6-diene-3,20-dione by dehydrogenation and subsequent reaction with Dimethylsulfoxonium methylide.

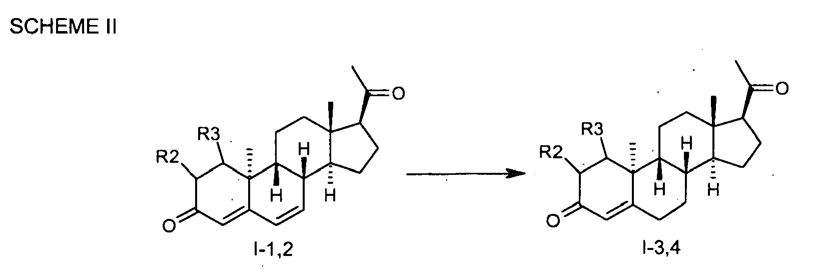

According to Scheme II, the optionally 1,2 methylene substituted 9β,10α-pregna-4,6-diene-3,20-dione of general formula I-1,2 is then converted to the corresponding 9β,10α-pregna-4-ene-3,20-dione (9β,10α-progesterone) of general formula I-3,4 under reducing conditions according to the procedures disclosed in US patent No. 3,555,053.

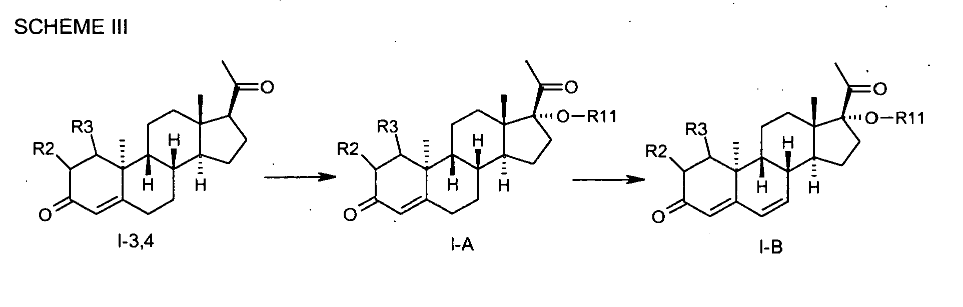

The introduction of the additional side chain in C17α position - as displayed in Scheme III - in order to obtain compounds of general formula (I-A), wherein R11 is -H, -(C₁-C₄)alkyl, or -CO-(C₁-C₄)alkyl, might be obtained by methods according to the procedures described by Halkes & van Moorselaar [1969] and within US patent Nos. 3,555,053 and 3,937,700 by introduction of a 17α-hydroxy group and optionally subsequent etherification or esterification of the hydroxyl group at carbon atom 17. If desired, in order to obtain compounds of general formula (I-B), the double bond in C6-C7 position can be reintroduced by dehydrogenation.

The functionalization of the C17 position starts with the introduction of a -OH group in C17 alpha position: For example, the (9β,10α)-pregna-4-ene-3,20-dione of formula I-3 or I-4 can be reduced by using a suitable reducing agent such as lithium aluminum hydride (LAH) to produce the corresponding 3,20-diol. The 3-hydroxy group is then selectively re-oxidized by means of a selective oxidizing agent such as 2,3-dichloro-5,6-dicyanonezoquinone (DDQ) in an aromatic solvent or manganese dioxide. The resulting 20-hydroxy-(9β,10α)-pregna-4-ene-3-one is further dehydrated by tosylation with tosyl chloride in pyridine. Subsequent treatment of the generated tosylate with boiling pyridine affords the 17,20 unsaturated derivative in a mixture of cis and trans isomers. The latter compound is then oxygenated using an amine oxide such as N-methylmorpholine-N-oxide (NMMO) as stoichiometric oxidizing agent and additional hydrogen peroxide in the presence of a catalytic amount of osmium tetroxide to produce the corresponding 17α-hydroxy-9β,10a-pregna-4-ene-3,20-dione.

This compound may be further modified by subjection to an etherification or esterification reaction at the hydroxyl group at the carbon atom C17, whereby the reactions are generally described within Belgian patent specification BE 577,615 or US patent No. 3,937,700. Suitable acylating agents are carboxylic acids, carboxylic acid anhydrides or carboxylic acid chlorides in the presence of a catalyst such as p-toluene sulphonic acid, trifluoroacetic acid, anhydride or pyridine-HCI or in the presence of an acid binder such as an organic base, for example, collidine. The acylation reaction is carried out in the presence of a solvent such as a hydrocarbon, for example, benzene or toluene. The reaction temperature may vary between room temperature and the boiling point of the solvent used. Since - if the starting material contains, apart from the 17-OH group, one or more further OH-groups - these will also be esterified, the further OH-groups have to be protected in advance. Alternatively, the alkylation reaction may be carried out by a reaction with an alkylhalide in the presence of Ag₂O, or by a reaction of dihydropyrane or dihydrofurane in a weak acidic, weak alkaline or neutral medium.

Finally, the obtained compounds might be again dehydrogenated to afford the 4,6 unsaturated derivative of general formula I-B.

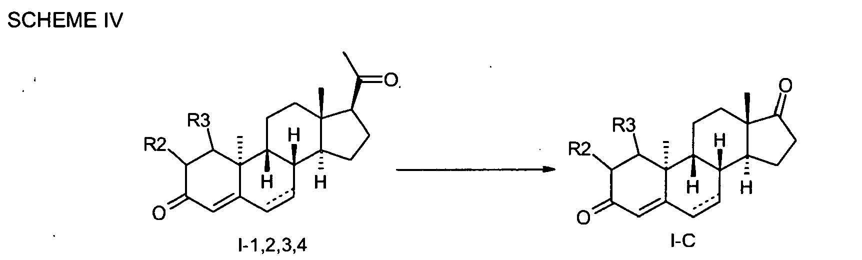

According to Scheme IV, the optionally 1,2 methylene substituted 9β,10α-pregna-4-(6-di)-ene-3,20-dione of general formula I-1,2,3,4 might be further converted to the corresponding 18-methyl-9β,10α-androst-4-(6-di)-ene-3,17-dione of general formula I-C by a sequence of reduction, oxidation and elimination followed by an ozonolysis of the intermediate 18-methyl-9β,10α-pregna-4,17(20)-diene-3-one or the intermediate 18-methyl-9β,10α-pregna-4,6,17(20)-triene-3-one as described by Halkes & van Moorselaar [1969].

### Process of the microbial 11β-hydroxylation

### General Performance

The process is carried out in the usual way. To this end, typically first a sterilized nutrient solution (=medium) is produced for the bacterial strain, and this nutrient solution is then inoculated with the bacterial strain, typically by scratching some colonies from an agar plate and suspending them into the medium, and subsequently cultivated. Optionally, a second preculture can be prepared by inoculating new nutrient solution with an aliquot of the culture suspension obtained. The preculture that is produced in this way is then added to a fermenter that also contains a suitable nutrient solution. Preferably after a growth phase for the culture of the strain, the starting substance - a compound of general formula I - is then added to the fermenter, so that the reaction according to the invention - the transformation of the compound of general formula I to the corresponding 11-hydroxylated compound of general formula IV - can proceed. After the reaction has been terminated, the mixture of substances is purified in the usual way or in a way according to the present invention to isolate the desired 11β-hydroxylated retrosteroid.

### Amycolatopsis mediterranei strains

The process provided by the present invention is based on the discovery that microorganisms of the species *Amycolatopsis mediterranei* are capable of introducing an 11β-hydroxy group into 11-unsubstituted 9β,10α-pregna-4,6-diene-3,20-dione and 9β,10α-pregna-4-ene-3,20-dione derivatives. These bacterial strains, obtainable from natural materials, such as soil and also available in public culture collections, can utilize retrosteroids as carbon source or are able to tolerate these steroids in the presence of other assimilable carbon sources.

Accordingly, the process provided by the present invention for the manufacture of the retrosteroids of formula IV or V as defined hereinbefore comprises fermenting a corresponding 11-unsubstituted retrosteroid with a member of the species *Amycolatopsis mediterranei.* Examples of specific *Amycolatopsis* mediterranei strains which are useful in carrying out the process of this invention include the newly identified strain LS30 as well as strains available from public culture collections such as DSM 43304 (corresponding to ATCC 13685, CBS 121.63, CBS 716.72, DSM 40501, IFO 13415, IMET 7651, ISP 5501, JCM 4789, KCC S-0789, LBG A 3136, NBRC 13142, NBRC 13415, NCIB 9613, NRRL B-3240, RIA 1376, VKM Ac-798), DSM 40773, and DSM 46096 (corresponding to ATCC 21411, IMET 7669). In one embodiment the strain *Amycolatopsis mediterranei* LS30 is the strain *Amycolatopsis mediterranei* as deposited under DSM 17416 at the DSMZ ("Deutsche Sammlung von Mikroorganismen und Zellkulturen").

Mutants of *Amycolatopsis mediterranei* strains generated by methods of molecular biology, by chemical methods (e g by treatment with a nitrite) or by physical methods (e g irradiation) can also be used in the process of the invention. Alternatively, the enzyme or enzymes which ferment the 11-unsubstituted retrosteroids can also be removed from the bacterial culture or the nutrient medium and brought into contact with the steroid substrate in the absence of the living cells. Preferably, the bacterial strains themselves are used in the practice of this invention to avoid additional manufacturing steps. The bacterial strains or enzymes isolated therefrom can be immobilized on a suitable substrate if desired.

The process of this invention is carried out under conditions customarily employed for 11β-hydroxylation with actinomycetes species; for example such as displayed within GB 1,111,320.

### Nutrient Solution (Nutrient Media)

The *Amycolatopsis mediterranei* strains used in the process of the invention can be cultivated on solid or liquid nutrient solutions (=media) which contain a source of assimilable nitrogen, a source of assimilable carbon and inorganic salts.

Suitable sources of assimilable nitrogen include animal, vegetable, microbial and inorganic compounds such as meat extracts, peptones, corn steep, yeast extracts, glycine and sodium nitrate, or mixtures thereof. Suitable sources of assimilable carbon include all sugars and polymers thereof (for example, starch, dextrin, saccharose, maltose and glucose) and amino acids, proteins, peptones, fatty acids, fats and steroids (especially retrosteroids) as well as mixtures thereof. Preferably, the nutrient medium contains yeast extracts in amounts of up to 2.5 % (w/w), preferably from 0.2 - 2 % as nitrogen source and glucose in amounts of up to 20 % (w/w), preferably from 5 - 10 % as carbon source.

The medium can contain trace elements (present naturally or added) which are available from mineral or organic ingredients. The presence of iron is especially desirable. Preferably, the nutrient medium contains iron in the form of Fe³⁺ ions in concentrations from 0 to 200 mg/ml (FeCl₃), preferably about 50 mg/ml FeCl₃. Sulphur can be present in the form of organic or inorganic compounds which are present in other components of the medium or can be specially added. The same is true for phosphorus, but in general it is present as an inorganic salt.

According to requirements or desire, further growth factors or stimulants such as vitamins (e.g. biotin or pyridoxin) or auxins (e.g. indolyl-acetic acid) can be added to the media.

In order to protect against infections, the medium can be sterilized and, in addition, can be provided with materials which inhibit the growth of e.g. bacteria.

For larger volumes of the culture medium, in particular in fermenters, an antifoam agent can be added to the medium. Optionally, the degree of foaming can be measured with an antifoam electrode and controlled by automatic addition of an antifoam agent. Antifoam agents comprise, for example, silicon-based antifoam agents or surfactants such as PEG or PPG.

### Culture conditions (pH value, temperature, incubation time)

Prior to inoculation, the pH value of the nutrient medium is desirably adjusted within the approximate range: The optimum pH value for the growth of *Amycolatopsis mediterranei* is from 5 to 8, preferably from 7.0 to 7.5. The cultivation temperature is preferably set to the optimal growth temperature of *Amycolatopsis mediterranei,* which is from 18°C - 40°C, preferably from 25 - 35°C, and most preferably from 28 - 32°C.

As a rule, however, *Amycolatopsis mediterranei* is allowed a certain pre-growth period before starting the microbial transformation process. Typically, the bacteria are cultivated for up to 96 hours, preferably from about 12 to about 72 hours, and even more preferred from about 24 to about 48 hours. However, the actual growth phase might vary depending on the volume and the age of preculture used for inoculation and depending on the volume of the culture medium. Preferably, the bacteria are in the middle or late phase of their exponential growth period when the transformation process is started by addition of the retrosteroid of formula (I).

A submerged fermentation technique can be employed. Preferably, the cultures are grown under aeration (i.e. by shaking the flasks containing the fermentation medium on a rotary shaker with a certain speed, or - in a fermenter - by stirring and pumping sterile air through the fermentation medium).

### Addition of the 11-unsubstituted retrosteroid (9β.10α-steroid) (= "educt" or "substrate")

The 11-unsubstituted retrosteroid of formula (I) can be added to the fermentation batch at any stage of growth of the *Amycolatopsis mediterranei.* Preferably, however, the *Amycolatopsis mediterranei* is allowed a certain pre-growth period as set out above, and the 11-unsubstituted retrosteroid is first added 12 to 96 hours after the beginning of fermentation. The 11-unsubstituted retrosteroid to be transformed can be added in any convenient manner, but preferably in such a way that a maximal contact surface between 11-unsubstituted retrosteroid and the bacteria results. For example, the 11-unsubstituted retrosteroid can be added to the culture as a powder by mechanical dispersion into the medium or in an emulsified form by means of dispersion agents or it can be added in solution dissolved in an organic solvent miscible with water (e g. acetone, propylene glycol, glycolmonomethyl ether, dimethyl sulphoxide (DMSO), dimethyl formamide and alcohols such as methanol or ethanol). Care must be taken, however, to keep the level of any such solvent used below that which adversely affects the bacteria, i.e. generally less than about 1 percent by volume. Emulsification of the 11-unsubstituted retrosteroid substrate can be performed, for example, by the latter being sprayed in micronized form or in a water-miscible solvent (such as methanol, ethanol, acetone, glycolmonomethyl ether, dimethylformamide or dimethyl sulfoxide) under strong turbulence into (preferably decalcified) water, which contains the usual emulsification auxiliary agents. Suitable emulsification auxiliary agents include, but are not limited to, nonionic emulsifiers, such as, for example, ethylene oxide adducts or fatty acid esters of polyglycols. Preferably the educt (the 11-unsubstituted retrosteroid) is dissolved in DMSO in a concentration of up to 40 mg/ml, and then added to the medium after sterilization under sterile conditions.

The concentration of the added 11-unsubstituted retrosteroid has a great influence on the yield and efficiency of the outcome of the fermentation reaction. Due to the relatively low solubility of the compounds of general formula (I) and (II) in aqueous media, the concentration of the educt and product should not exceed a certain value. Depending on the age of the bacterial culture and of the solubility of the educt used up to about 1 gram thereof can be added per liter of nutrient solution; however, preferably about 50 mg to about 500 mg of the educt should be added per liter nutrient solution, and even more preferably between 100 - 250 mg per liter. Mostly preferred, the amount of the steroidal starting compound should not exceed values of 150 mg per liter fermentation medium. Correspondingly, care should also be taken, that the amount of the desired hydroxylated product should not accumulate above values of 1 g per liter of nutrient solution; preferably the product should be present in amounts from about 50 mg to about 500 mg per liter and even more preferably between 100 - 250 mg per liter fermenter solution. Mostly preferred, the amount of the hydroxylated product should not exceed values of 150 mg per liter fermentation solution.

In one embodiment, the whole amount of the 11-unsubstituted retrosteroidal compound is added at once at the beginning of the transformation process or over a period of 1 to 5 hours (i.e.-a short time period) at the beginning of the transformation process.

In an alternative embodiment, the amount of the 11-unsubstituted retrosteroidal compound is added in a continuous way over the complete transformation period. For example, the 11-unsubstituted retrosteroid is added in a concentration from 1 to 20 mg per hour of cultivation and per liter of nutrient solution (mg/h/l). Preferably, the 11-unsubstituted retrosteroid is added in a concentration from 2 to 5 mg per hour of cultivation and per liter of nutrient solution (mg/h/I).

### Transformation conditions

The transformation is normally carried out at the same pH and temperature which the *Amycolatopsis mediterranei* strain requires for growth. However, in some instances, the optimum temperature and/or pH for growth may not be optimal throughout for the hydroxylation of the 11-unsubstituted retrosteroids and needs to be adapted. The time necessary for the transformation of the 11-unsubstituted retrosteroid fluctuates somewhat, depending on fermentation batch, mode of opera- .. tion and individual Amycolatopsis mediterranei strain used, but generally lies within the approximate period from 2 to 172 hours. Preferably, the transformation time lies from about 12 to 96 hours, and even more preferred from 36 to 60 hours.

The fermentation process is typically carried out under aerobic conditions, preferably the relative O₂ saturation pO₂/pO_{2,max} of the medium is regulated to be from about 5% to about 95%, preferably from about 20% to about 80%, even more preferred from about 30% to about 75%, and most preferred from about 40% to about 70%.

The course of the transformation can be determined for each fermentation batch by means of the usual analytical methods, such as thin layer chromatography, ultraviolet absorption or HPLC (high performance liquid chromatography) of small probes taken from the fermentation batch. Furthermore, the course of the transformation process is monitored by analysis of parameters such as pH value, temperature, relative O₂ saturation, glucose content etc.

Specific details of the growth phase and transformation process to provide a particular 11β-hydroxy-retrosteroid of general formula IV or V are provided in the example section hereinbelow. The optimum substrate concentration, time of substrate addition and duration of transformation are dependent upon the structure of the 11β-unsubstituted retrosteroids of formula I, II or III and eventually also upon the individual *Amycolatopsis mediterranei* strain used for the hydroxylation reaction. These variables can be readily determined in each individual hydroxylation reaction with routine preliminary experimentation within the expertise of one of ordinary skill in the art.

### Induction

The rate of the transformation and the yield of product can be increased by inducing the desired enzymes prior to the addition of 11-unsubstituted retrosteroid. Any steroid including normal steroids such as estradiol and testosterone can be used as enzyme-inducers. However, those steroids which are more water soluble than the substrate to be employed are preferred enzyme-inducers. The amount and time of addition of the inducers are not critical, although from about 1 to 10 weight per cent of the steroid employed is normally added to the medium.

A further possibility for increasing the yield of 11β-hydroxy retrosteroids of formula IV or V consists in the addition of cytotoxic substances such as 2,4-dinitro-phenol or potassium cyanide or antibiotic substances such as chloromycetin to the fermentation batch in which the enzymes necessary for the 11β-hydroxylation are already present.

### Isolation of the hydroxylated retrosteroidal product

After complete transformation, the hydroxylated retrosteroid is isolated from the batch. Preferably, the fermentation batch is first of all separated into supernatant and cellular material, bacterial debris and other solid materials by known separation means such as sedimentation and decanting, separation, filtration or centrifugation, which methods are explained in more detail below. A possible method for effecting isolation is extraction by means of a solvent for steroids which is immiscible with water (e.g. ethyl acetate, methylene chloride, chloroform, methyl isobutyl ketone, carbon tetrachloride, ether, trichloroethylene, alcohols, benzene and hexane). The cellular substance can also be separated with the solvents mentioned in the preceding paragraphs and also extracted by means of water-miscible solvents (e.g. acetone, dimethyl sulphoxide and ethanol). The steroids obtained from the extracts can be purified by recrystallization, chromatography or by means of countercurrent distribution and separated from the undesired by-products of the fermentation process.

Preferably, the 11β-hydroxy-retrosteroids of formula IV or V are isolated from the fermentation batch by column chromatography using a semi-polarnonionic adsorber resin. A similar protocol for the isolation of conjugated estrogens from pregnant mares' urine (PMU) was disclosed in international patent application WO 98/08526 and was adapted for the purpose of the present invention.

In the first step the fermentation broth is depleted from any solids, bacterial cells and components and any mucilaginous substances in a known manner. Advantageously, the solid materials are separated by known separation methods, for instance decanting, separation and/or filtration. Thus the fermentation broth can for instance be passed through a known separating apparatus, e.g. a separator, a filtration unit or a sedimenter. Commercially-available separators may serve as a separating apparatus, e.g. nozzle separators or chamber separators may be used. If desired, a microfiltration apparatus or an ultrafiltration apparatus may also be used, and if they are used it is possible to obtain a substantially bacteria-free and filtered supernatant at the same time. Alternatively, centrifugation can be used as separation means.

The supernatant of step a), a concentrate obtained therefrom by reducing its volume or a retentate obtained therefrom by membrane filtration can be used as the starting supernatant material for the purification method according to the invention.

If a concentrated supernatant retentate is to be used instead of the supernatant, this may be obtained from the supernatant by known membrane filtration. The solids content of the retentate and the composition thereof may vary according to the individual fermentation culture used and the membrane used for membrane filtration, for instance the pore diameter thereof, and the conditions of the filtration. For instance, when using a nanofiltration membrane, a practically loss-free concentration of the steroid content in the supernatant retentate can be achieved with simultaneous removal of up to 50% by weight of the lower-molecular weight fermentation broth contents. Supernatant retentates which have been concentrated up to a ratio of approximately 1:10, for instance a ratio of about 1:7, and the volume of which can thus be reduced to approximately 1/10, for instance about 1/7, of the original supernatant volume, can be used for the method according to the invention.

Suitable adsorbents for use for the purification method according to the invention are polymeric adsorption resins. Particularly preferred adsorption resins are semipolar, in particular non-ionic semipolar, polymeric adsorption resins. The non-ionic semi-polar polymeric adsorber resins which can be used in method step b) are porous organic non-ionic polymers which, in contrast to nonpolar hydrophobic polymeric adsorber resins, have an intermediate polarity (e.g. with a dipole moment of the active surface of the resin in the range of 1.0 to 3.0, in particular 1.5 to 2.0 Debye) and a somewhat more hydrophilic structure, for example polycarboxylic acid ester resins. Advantageously, macroporous semi-polar resins having a preferably macroreticular structure and average pore diameters in the range of 50 to 150, preferably 70 to 100 Angstrom, and a specific surface area in the range of 300 to 900 m²/g, preferably in the range of 400 to 500 m²/g, are used. Macroporous cross-linked aliphatic polycarboxylic acid ester resins, in particular cross-linked polyacrylic ester resins such as Amberlite XAD-7^{™} or Amberlite XAD-7-HP^{™} manufactured by Rohm and Haas, have proved particularly suitable.

According to the invention, the adsorption of the hydroxylated retrosteroids on the semi-polar adsorber resin can be effected by contacting the supernatant or the concentrate or retentate thereof with the adsorber resin, in that the supernatant material is introduced into a reactor containing the adsorber resin and is kept in contact with the adsorber resin therein for a sufficient time for adsorption of the steroid content. Once adsorption of the 11β-hydroxy substituted retrosteroids on the semi-polar adsorber resin has taken place, the adsorber resin laden with the 11β-hydroxy substituted retrosteroids can be separated from the rest of the supernatant material in known manner. Advantageously, the supernatant material can be passed through a column containing the adsorber resin at such a flow rate that the contact time is sufficient for adsorption of the steroid content. Suitable flow rates are for instance those which correspond to a throughput of 3 to 10, preferably 5 to 7, parts by volume of supernatant material per one part by volume of adsorber resin per hour. The adsorption is preferably effected at room temperature. Advantageously, the rate of supernatant material flow through the reactor can be controlled by operating at a slight overpressure or underpressure (i.e. relative to ambient pressure). The quantity of non-ionic semi-polar adsorber resin to be used may vary depending on the type of adsorber resin used and the quantity of solids contained in the supernatant material. When using supernatant, for instance one part by volume adsorber resin, e.g. cross-linked aliphatic polycarboxylic acid ester adsorber resin, can be loaded or charged with up to 80, preferably from about 30 to 50 parts by volume pretreated supernatant, without perceptible quantities of steroidal compounds being detectable in the effluent. When using a supernatant concentrate or supernatant retentate, the loading capacity of the adsorber resin is of course reduced to the extent which the supernatant material has been concentrated. For instance, 1 part by volume cross-linked aliphatic polycarboxylic acid ester adsorber resin may be laden with a quantity of concentrated supernatant material corresponding to 20 to 80, preferably 30 to 50, parts by volume non concentrated supernatant.

The semi-polar adsorber resin laden with the 11β-hydroxy substituted retrosteroids is washed in method step c) with washing water adjusted to a pH range of at least 12.0, in particular of 12.5 to 14, preferably about 13.5 to 14. Aqueous solutions of inert basic substances which are soluble in the supernatant and which are strong enough to reach a pH value of at least 12.5 can be used as washing liquid. Suitable water-soluble basic substances which are inert to the semi-polar polymeric adsorber resin are preferably water-soluble inorganic bases such as alkali metal or alkaline-earth metal hydroxides, in particular sodium hydroxide. Advantageously, the washing water only contains about that quantity of basic substances which is required to achieve the desired pH value, preferably approximately pH 13 to 14. The quantity of alkaline washing water is selected such that it is sufficient to substantially remove all other contents of the fermentation broth, without significant quantities of 11β-hydroxy substituted retrosteroids being washed out with them. For instance, the use of 2 to 10, in particular 4 to 6, bed volumes washing liquid per bed volume adsorber resin has proved advantageous. In this case, the washing water is advantageously passed through a reactor containing the adsorber resin at a throughput rate of 3 to 10, preferably 5 to 7, parts by volume of washing water per one part by volume of adsorber resin per hour.

In method step d), the non-ionic semipolar adsorption resin laden with the 11β-hydroxy substituted retrosteroids is then optionally washed with water in a second intermediate washing operation following process step c). The amount of washing water is individually adapted. Preferably, the use of 2 to 10, more preferably 4 to 6, bed volumes washing water per bed volume adsorption resin has proved expedient. In this case, the washing water is expediently passed through a reactor containing the adsorption resin at a through flow rate of 3 to 10, preferably 5 to 7, parts by volume washing water per 1 part by volume adsorption resin per hour.

In an advantageous embodiment of the method according to the invention, the optional washing step d) is carried out at temperatures below room temperature, particularly at temperatures between 0°C and 10°C, since it has been shown that losses of the 11β-hydroxy substituted retrosteroid possibly due to the additional intermediate washing operation can be considerably reduced. Usually the ambient temperature is regarded as "room temperature", e.g. the term designates a temperature of between 20° and 30°C. It is very expedient to perform the method at temperatures of actually 0°C or approximately 0°C. In practice, it is therefore recommended to operate at temperatures of close to but above 0°C and to ensure that the aforementioned temperature ranges are maintained by suitable measures. Conventional measures for lowering the temperature may be used for this, e.g. the use of cooled reactors, cooled materials and/or cooled starting materials such as the supernatant material. From practical points of view a temperature range from 0°C to about 5°C, in particular of 0°C to about 3°C, can be considered as temperatures of 0°C or of approximately 0°C.

In order to keep any losses of the 11β-hydroxy substituted retrosteroidal compound during the intermediate washing as low as possible, according to this variant of the process the washing water used in the intermediate washing operation and/or also the washing water which has been rendered alkaline used in process step c) will be precooled to temperatures below room temperature, in particular to temperatures between 0°C and 10°C. Further expedient or preferred temperature ranges are, as stated above, temperatures of 0°C to about 5°C, in particular of from 0°C to about 3°C. Preferably operation is at temperatures of 0°C or of approximately 0°C, i.e. preferably the washing water used in the intermediate washing operation and/or also the washing water which has been rendered alkaline used in process step d) is precooled to temperatures close to but above 0°C. By the use of cooled washing water which has been rendered alkaline in process step c), a type of precooling or maintaining of the cooling of the adsorption resin which has already taken place is achieved, e.g. in order to prevent undesirable reheating of the water from taking place when using cooled washing water for the intermediate washing. Preferably therefore the intermediate washing step and the process step c) are both carried out in the temperature range, e.g. at temperatures below room temperature, in particular at temperatures between 0°C and 10°C, or preferably in the same temperature ranges as stated above.

In the above variant of the process, in which the method is carried out at temperatures below room temperature, it may be desirable to use all devices used, such as reactors for receiving the semipolar adsorption resin or reactors already containing same and/or the supernatant used, precooled accordingly to temperatures below room temperature, in particular to temperatures between 0°C and 10°C, or to the preferred temperature ranges given above.

In method step e), the washed adsorber resin laden with the 11β-hydroxy substituted retrosteroids is then treated with a quantity of an elution liquid sufficient for elution of the 11β-hydroxy substituted retrosteroids, and in method step f) then an eluate containing the 11β-hydroxy substituted retrosteroids is obtained. The elution liquid used according to the invention preferably consists essentially of a water-miscible organic solvent selected from the group consisting of water-miscible ethers, lower alkanols and lower aliphatic ketones or a mixture of such a water-miscible organic solvent and water which has optionally been rendered alkaline. Suitable ether constituents of the elution liquid include water-miscible cyclic ethers such as tetrahydrofuran or dioxane, but also water-miscible open-chain ethers such as ethylene glycol dimethyl ether (= monoglyme), diethylene glycol dimethyl ether (= diglyme) or ethyloxyethyloxy ethanol (= Carbitol). Suitable lower alkanols include water-miscible alkyl alcohols with 1 to 4, preferably 1 to 3, carbon atoms, in particular ethanol or isopropanol. Suitable lower aliphatic ketones include water-miscible ketones with 3 to 5 carbon atoms, in particular acetone. Elution liquids in which the organic solvent is ethanol have proved particularly advantageous. Advantageously, mixtures of one of the aforementioned water-miscible organic solvents and water which has optionally been rendered alkaline are used as elution liquids. The pH value of such water-containing eluents is in the neutral to alkaline range up to pH 13 and may advantageously be approximately 10 to 12. A solvent which is stable in the pH range used is selected as the solvent component in the water-containing elution liquid. In water-containing alkaline elution liquids having pH values of approximately 10 to 12, lower alkanols, preferably ethanol, are particularly suitable as solvent components. The desired pH value of the water-containing eluent is achieved by adding a corresponding quantity of a water-soluble inert basic substance, preferably an inorganic base, for instance an alkali metal or alkaline earth metal hydroxide, in particular sodium hydroxide. In water-containing elution liquids there may be a volume ratio of water-miscible organic solvent to water in the range of 40:60 to 20:80, preferably approximately 30:70. The quantity of eluent used may be approximately 3 to 10, in particular approximately 4 to 6, bed volumes of elution liquid per bed volume of adsorber resin. Advantageously, the elution liquid is passed through a reactor containing the adsorber resin laden with the 11β-hydroxy substituted retrosteroid at such a flow rate that the contact time is sufficient for complete elution of the 11β-hydroxy substituted retrosteroids. When using a mixture of ethanol with water in a volume ratio of 30:70, for instance flow rates of 3 to 10, preferably 5 to 7, parts by volume elution liquid per 1 part per volume adsorber resin per hour are suitable. When using ethanol as elution liquid, for instance flow rates of 3 to 10, preferably 5 to 7, parts by volume elution liquid per 1 part per volume adsorber resin per hour are suitable.

Advantageously, the elution is performed at a temperature in the range from room temperature to approximately 60°C, preferably at room temperature. If desired, the flow rate is regulated by operating at slightly elevated pressure, e.g. at an overpressure of up to 0.2 bar (relative to ambient pressure), and the eluate is collected in several fractions. The content of the desired 11β-hydroxy substituted retrosteroid and optionally the content of the corresponding 11 unsubstituted retrosteroids in the individual eluate fractions may be determined in known manner by HPLC.

Upon elution, a practically steroid-free preliminary fraction is initially obtained, the quantity of which generally corresponds to approximately one bed volume. In case that the intermediate washing step e) was performed, already the first fraction obtained when starting the elution process can already contain significant amounts of the 11β-hydroxy substituted retrosteroid. However, the bulk of the 11β-hydroxy substituted retrosteroids, for instance between 80 and 99% of the 11β-hydroxy substituted retrosteroids present in the starting supernatant, is in the subsequent main eluate fractions, the quantity of which is generally 2 to 4 bed volumes. Generally only traces of steroids are contained in the subsequent afterrun fractions. If succeeding fractions are obtained which still have a significant steroid content, these may be combined with the 11β-hydroxy substituted retrosteroid containing main eluate for further processing.

The adsorber column can be thereafter regenerated by washing with 5 to 10 bed volumes of water.

The main eluate separated from the adsorber resin in the manner previously described contains the 11β-hydroxy substituted retrosteroids. If desired, the volume of the eluate may be further reduced in known manner, e.g. by evaporation, in order to obtain a concentrate substantially freed of organic solvent. Typically, the 11β-hydroxy substituted retrosteroid precipitates from the concentrated eluate. Alternatively, the volume of the eluate concentrate can be further reduced until the 11β-hydroxy substituted retrosteroids are obtained as solid material. Typically, the precipitated retrosteroidal compound is isolated by filtration and can be further washed with water. After drying, the solid material can be directly used for subsequent transformation reactions without need for further purification. However, if desired, the compound obtained can be again dissolved in a suitable organic solvent and crystallized therefrom. Alternatively, further purification of the compound can be achieved by flash chromatography.

### 11β-hydroxy-9β,10α-steroids (11β-hydroxy-retrosteroids) of formula IV: Yield, identification and use

The yield of the 11β-hydroxy-retrosteroids of formula IV obtained by the process of the invention will vary depending upon the incubation and fermentation conditions employed, but generally, the yield of the desired product at the end of the fermentation process is greater than about 40%, preferably greater than about 50%, even more preferred greater than 60%, and most preferred more than 70% of the theoretical yield based on the amount of starting material. A person skilled in the art can, with routine experimentation, select the optimum conditions, including the choice of the best bacterial strain and the optimum substrate concentration for a given starting material, to provide optimum yield.

Including the preferred isolation process of the 11β-hydroxy-retrosteroids of formulas IV and V from the fermentation batch and the additional purification steps using flash chromatography, the overall yield of the 11β-hydroxy-retrosteroids of formula IV and V lies from about 40% to about 60% of the theoretical yield based on the amount of starting material.

Separation and identification of the fermentation products (i.e. the 11β-hydroxy retrosteroids of formulas IV and V) can be effected by thin layer chromatography. A suitable color reaction for identification is achieved by spraying with concentrated sulphuric acid followed by 3 per cent vanilline in ethanol. The various steroid products give with sulphuric acid a color reaction on heating. The coloring with the vanilline in ethanol spray is likewise characteristic. Alternatively, the fermentation products can be identified and also quantified by HPLC (high performance liquid chromatography) analysis.

The 11β-hydroxy-retrosteroids of formulas IV and V obtained by carrying out the fermentation step _ of the process represent preferred intermediates for the synthesis of novel retrosteroidal compounds with substituents in the 11-position which show progestational and/or anti-progestational activity.

The following examples are intended to illustrate the invention in further detail.

### EXPERIMENTAL SECTION

### Experimental

### 1. Synthesis of Educts (not forming part of the invention)

1.1. Synthesis of 1,2-Methylene-9β,10α-pregna-4,6-diene-3,20-dione 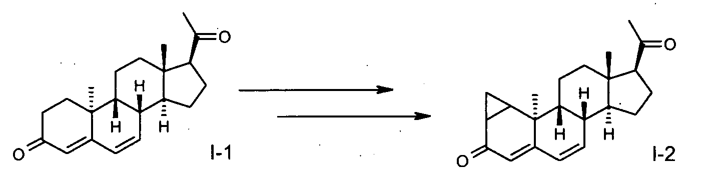 Commercially available Dydrogesterone (9β,10α-Pregna-4,6-diene-3,20-dione) of formula I-1 is converted into the corresponding 1,2-Methylene-9β,10α-pregna-4,6-diene-3,20-dione ((1,2-Methylene-dydrogesterone)) of formula 1-3 by dehydrogenation and subsequent reaction with Dimethylsulfoxonium methylide as described within US patent No. 3,937,700.
1.2. 9β,10α-Pregna-4-ene-3,20-dione (9β,10α-Progesterone) 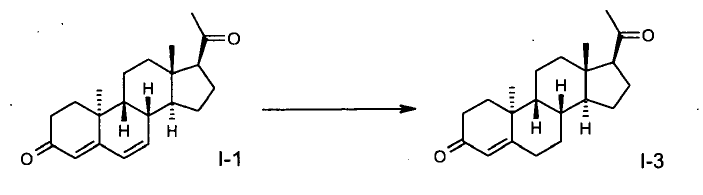
   Commercially available Dydrogesterone (9β,10α-Pregna-4,6-diene-3,20-dione) of formula I-1 is converted to the corresponding 9β,10α-Pregna-4-ene-3,20-dione (9β,10α-Progesterone) of formula 1-3 under reducing conditions.
   A suspension of 0.75 g of Pd/CaCO₃ (5% Pd) in 100 ml of toluene was hydrogenated with H₂. Then a solution of 50 g of Dydrogesterone (160 mmol) in 550 ml of toluene was added; residues of Dydrogesterone were added by rinsing with 2x 50 ml portions of toluene. The hydrogenation was carried out under vigorous stirring until 3.6 I of H₂ have been absorbed (approx. 1 h). The suspension was suction filtered through diatomaceous earth and rewashed with some toluene. The solvent was removed under a vacuum, and the resulting residue redissolved in approx. 90 ml of dichloromethane (DCM). The product was crystallized by addition of 900 ml of warm hexane. The crystals formed were removed by suction filtration and rewashed with 100 ml of 10% DCM/hexane. Vacuum-drying gave rise to 36.9 g of (I-3) ([α]D₂₀ = -60 (c=1, CHCl₃)). The solvent was completely removed from the mother liquor and the residue (approx. 13 g) was dissolved in approximately 20 ml of DCM. Crystallization was initiated by addition of 150 ml of hexane. After suction filtration and washing, 7.3 g of secondary crystals of (I-3) were obtained. Overall yield: 44.2 g of (I-3) (88%).
1.3. Synthesis of 17α-Ethoxy-9β,10α-pregna-4,6-diene-3,20-dione 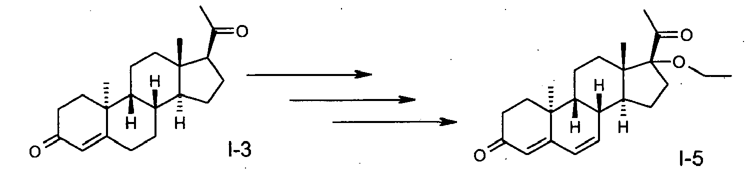 9β,10α-Pregna-4-ene-3,20-dione (9β,10α-Progesterone) of formula I-3 obtained from Example 1.2 is then converted into the corresponding 17α-Ethoxy-9β,10a-pregna-4-ene-3,20-dione of formula 1-5 by a multi-step reaction as described in the general section.
1.4. Synthesis of 17α-Ethoxy-1,2-methylene-9β,10α-pregna-4,6-diene-3,20-dione 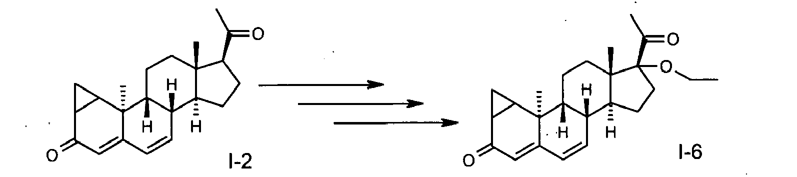 1,2-Methylene-dydrogesterone (obtained in Example 1.2) of formula I-2 is converted into the corresponding 17α-Ethoxy-1,2-methylene-9β,10α-pregna-4-ene-3,20-dione of formula 1-6 according to the protocols displayed in Example 1.2 and 1.3 hereinabove.
1.5. Synthesis of 18-methyl-9β,10α-androst-4-ene-3,17-dione 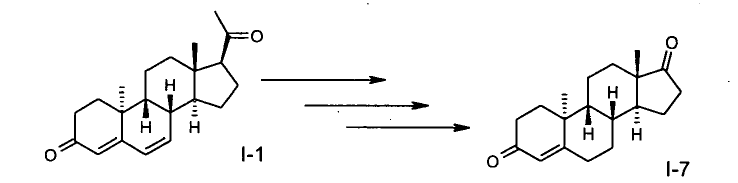 Commercially available Dydrogesterone (9β,10α-Pregna-4,6-diene-3,20-dione) of formula I-1 is converted into the corresponding 18-Methyl-9β,10α-androst-4-ene-3,17-dione (Des-acetyl-9β,10α-progesterone) of formula I-7 by a sequence of reduction, oxidation and elimination followed by an ozonolysis of the intermediate 18-methyl-9β,10α-pregna-4,17(20)-diene-3-one as as described in the general section.

### 2. Biotransformation with Amycolatopsis mediterranei

### Detection of the Educt and Product

The course or the result of the fermentation reaction can be monitored by HPLC analysis of probes taken from the fermentation medium or of diluted probes taken after extraction of the product.

The chromatography was performed using an apparatus (from Shimadzu) equipped with a LC-10 AT VP pump, a SPD-10 A VP UV-VIS wavelength detector, a SCL-10 A VP control system, a FCV-10 10 AL VP solvent organizer and a SIL-10 AD-VP auto sampler. The steroids and transformation products were separated using a C18 reversed-phase ET 250/4 Nucleosil 120-5 column (Machery & Nagel) and a solvent system of methanol / water (75:25, by volume). Operating conditions were a sample volume of 20 µl, a flow rate of 0.5 ml/min, UV detection at 298 nm and a pressure of about 92 bar.

### Nutrient medium

For the cultivation of *Amycolatopsis mediterranei* an aqueous medium containing 80 g/l glucose, 2 g/l yeast extract, 1.3 g/l K₂HPO₄x 3 H₂O and 1 g/l MgSO₄x 7 H₂O was used. The pH value was adjusted to pH 7.0 - 7.2 by means of 0.1 M HCl. Larger volumes (i.e. above 5 I) were not pH adjusted; the pH value of the medium was about 7.5 without adjustment. The media were sterilized at 121°C for at least 20 minutes (depending on volume size).
2.1. Biotransformation of Dydrogesterone with *Amycolatopsis mediterranei* LS30 (small culture volume)
*Amycolatopsis mediterranei* LS30 bacterial colonies were transferred from agar plates and grown in 500 ml Erlenmeyer flasks using 100 ml of the nutrient medium. The culture was incubated on a rotary shaker operating at 200 rpm and 30°C (Certomat, B. Braun, Germany). After 3 - 4 days of culturing under these conditions, the so-obtained preculture which contained the bacteria in form of pellets (i.e. in aggregated form) was gently homogenized by use of a stomacher. A 1-10% (vol/vol) inoculum, typically a 2% (vol/vol) inoculum, of the homogenized preculture medium was used to seed 500 ml flasks containing 100 ml of the same nutrient medium. The culture was again incubated as described hereinabove for 46 - 50 hours. Then, 15 mg of Dydrogesterone were added as a 20 mg/ml solution in DMSO to the second culture medium such that the initial concentration of the steroid was 0.015% (by weight). The resulting suspension was incubated at 30°C and 200 rpm for 46 - 50 hours as described hereinabove to promote hydroxylation of the Dydrogesterone. Then, the culture medium was centrifuged (20 min at 17,000 x g). Aliquots of the supernatant were analyzed by HPLC. The supernatant was extracted twice with ¼ volume of ethyl acetate under addition of solid NaCl. The organic phases were combined, dried over NaSO₄, and analyzed by HPLC as set out above. The results from three independent experiments are shown in the following table 1:

**Table 1: Yield of the fermentation of Dydrogesterone in Erlenmeyer flasks**

| | **1. Flask** | **2. Flask** | **3. Flask** |
|---|---|---|---|
| Amount Dydrogesterone added [mg] | 15 | 15 | 15 |
| 11OH-Dydrogesterone in the supernatant [mg] | 12.8 | 9.6 | 11.5 |
| Yield of the product | 81.2% | 60.9% | 72.9% |
| 11OH-Dydrogesterone in the organic phase after extraction [mg] | 9.6 | 7 | 9.4 |
| Yield of the extraction | 75.0% | 72.9% | 81.7% |
| Overall yield | 60.9% | 44.4% | 59.6% |

2.2. Biotransformation of Dydrogesterone with *Amycolatopsis mediterranei* LS30 (large fermenter batch)
*Amycolatopsis mediterranei* LS30 bacterial colonies were transferred from agar plates and grown in a 500 ml Erlenmeyer flask using 100 ml of the nutrient medium. The cultures were incubated on a rotary shaker operating at 200 rpm and 30°C. After 4 days of culturing under these conditions, 1 ml aliquots of the first preculture were used to seed two 500 ml flasks containing 80 ml of the nutrient medium (= inoculation culture). The cultures were again incubated as described hereinabove for 28 - 32 hours. Then, the 1 day old precultures were combined and added to the sterile fermenter (size: 15I, B. Braun, Germany) filled with 6 - 8I of the sterilized nutrient medium - the inoculation volume was set to about 2% vol/vol of the fermenter volume. The bacteria were allowed to grow for 40 - 44 hours with optional addition of PPG 2000 as an antifoaming agent at 30°C with aeration (3 I/min in the 15 I fermenter) and stirring (300 rpm in the 15 I fermenter at the beginning). The stirrer speed was optionally adjusted to higher speeds in order to obtain a relative O₂ saturation (pO₂/pO_{2,max}) of at least 50%. Then the addition of Dydrogesterone was started: Either the Dydrogesterone was added continuously over the following transformation period in the form of a 20 mg/ml solution in DMSO at a concentration of generally about 3 - 3.5 mg per liter of nutrient medium per hour of cultivation until the predetermined amount of Dydrogesterone was added (see table 2 for exact values) and then transformation was stopped, or the total amount of Dydrogesterone was added within 1 hour and then transformation was continued for 46 - 50 hours. After a fixed time period, the transformation was stopped and the bacteria were separated from the fermentation broth by microfiltration. The obtained supernatant was analyzed by HPLC. The hydroxylated Dydrogesterone was isolated from the supernatant as described below in Example 3. The results from four independent fermentations are summarized in the following table 2:

**Table 2: Yield of the fermentation of Dydrogesterone**

| | **1.** | **2.** | **3.** | **4** |
|---|---|---|---|---|
| Fermentation medium volume | 6.6 l | 8 l | 8.2 l | 8 l |
| Variable transformation conditions: | | | | |
| - Add. of Dydrogesterone - Stirrer velocity [rpm] - O₂/ pO_{2,max} | 23.4 mg/h 300-340 > 50% | 25.3 mg/h 290-360 > 50% | 25.5 mg/h 300 > 40% | (all in 1 h) 290-420 > 50% |
| Total amount of Dydrogesterone added [g] | 1.12 | 1.22 | 1.19 | 1.2 |
| Total amount 11OH-Dydrogesterone in the supernatant [g] | 0.86 | 0.79 | 0.83 | 0.77 |
| Yield of the product | 73.0% | 61.9% | 66.4% | 61.0% |

2.3. Biotransformation of 17-Ethoxy-1,2-methylene-dydrogesterone with Amycolatopsis mediterranei LS30
A) Fermentation in a 500 ml flask
   In an analogous manner to the procedure described in Example 2.1 using 15 mg 17-Ethoxy-1,2-methylene-dydrogesterone as educt, there was obtained 4 mg of 17-Ethoxy-11-hydroxy-1,2-methylene-dydrogesterone (Yield: 26%).
   ¹H NMR (501 MHz, CHLOROFORM-d): δ ppm 0.87 (s, 3 H) 0.89 - 0.93 (m, 1 H) 1.13 - 1.18 (m, 3 H) 1.30 - 1.36 (m, 1 H) 1.39 (s, 3 H) 1.40 - 1.49 (m, 1 H) 1.69 - 1.83 (m, 3 H) 1.87 - 1.94 (m, 1 H) 1.95 - 2.01 (m, 1 H) 2.11 - 2.17 (m, 4 H) 2.39 - 2.47 (m, 1 H) 2.49 - 2.55 (m, 1 H) 2.59 (dd, J=14.5, 4.4 Hz, 1 H) 2.70 - 2.77 (m, 1 H) 2.98 - 3.06 (m, 1 H) 3.39 - 3.47 (m, 1 H) 4.70 - 4.75 (m, J=2.4 Hz, 1 H) 5.51 - 5.53 (m, 1 H) 6.08 -6.10 (m, 2 H)
   ¹³C NMR (126 MHz, CHLOROFORM-d): δ ppm 13.8 (q, 1 C) 15.6 (q, 1 C) 16.4 (q, 1 C) 23.3 (t, 1 C) 24.2 (t, 1 C) 25.4 (d, 1 C) 26.5 (q, 1 C) 26.7 (d, 1 C) 28.6 (q, 1 C) 34.9 (d, 1 C) 36.9 (s, 1 C) 37.9 (t, 1 C) 44.2 (d, 1 C) 47.6 (s, 1 C) 48.1 (d, 1 C) 59.9 (t, 1 C) 69.3 (d, 1 C) 95.8 (s, 1 C) 120.3 (d, 1 C) 127.2 (d, 1 C) 139.0 (d, 1 C) 156.2 (s, 1 C) 198.3 (s, 1 C) 210.3 (s, 1 C)
B) Fermentation in a 15 I fermenter
   In an analogous manner to the procedure described in Example 2.2, 17-Ethoxy-1,2-methylene-dydrogesterone was used as educt in order to obtain the corresponding 17-Ethoxy-11-hydroxy-1,2-methylene-dydrogesterone derivative:

*Amycolatopsis mediterranei* LS30 bacterial colonies were transferred from agar plates and grown in a 500 ml Erlenmeyer flask using 100 ml of nutrient medium. The cultures were incubated on a rotary shaker operating at 200 rpm and 30°C. After 4 days of culturing under these conditions, 1 ml aliquots of the first-preculture medium were used to seed two 500 ml flasks containing 80 ml of the nutrient medium (= inoculation culture). The cultures were again incubated as described hereinabove for 24 - 30 hours. Then, the 1 day old precultures were combined and added to the sterile fermenter (size: 15 I) filled with 8 l of the sterilized nutrient medium. The bacteria were allowed to grow for 44 - 50 hours with optional addition of PPG 2000 as an antifoaming agent at 30°C with aeration (3 I/min) and stirring (290 rpm). The stirrer speed was adjusted to higher speeds in order to obtain a relative O₂ saturation (pO₂/pO₂,ₘₐₓ) of at least 50%. Then, 0.578 g 17-Ethoxy-1,2-methylene-dydrogesterone were added continuously in the form of a 8 mg/ml solution in DMSO at a concentration of generally about 22.7 mg per hour of cultivation. After 24 - 28 hours of fermentation, the process was stopped and the bacteria were separated from the fermentation broth by microfiltration. The obtained supernatant was analyzed by HPLC. The supernatant contained 0.187 g of 17-Ethoxy-11-hydroxy-1,2-methylene-dydrogesterone, corresponding to a yield of 31%. The 17-Ethoxy-11-hydroxy-1,2-methylene-dydrogesterone was isolated from the supernatant according to the procedure as described below in Example 3.
2.4. Biotransformation of 17-Ethoxy-dydrogesterone with Amycolatopsis mediterranei LS30
In an analogous manner to the procedure described in Example 2.1 using 10, 15 or 20 mg 17-Ethoxy-dydrogesterone as educt, the corresponding 17-Ethoxy-11-hydroxy-dydrogesterone was obtained in a yield of 32, 37 and 30%, respectively.
¹H NMR (400 MHz, CHLOROFORM-d): δ ppm 0.86 (s, 3 H) 1.15 (t, J=6.9 Hz, 3 H) 1.28 (s, 3 H) 1.37 - 1.52 (m, 1 H) 1.64 - 1.94 (m, 5 H) 2.15 (s, 3 H) 2.27 - 2.68 (m, 6 H) 2.70 - 2.79 (m, 1 H) 2.97 - 3.07 (m, 1 H) 3.39 - 3.52 (m, 1 H) 4.48 - 4.54 (m, 1 H) 5.70 - 5.73 (m, 1 H) 6.18 - 6.26 (m, 2 H)
¹³C NMR (101 MHz, CHLOROFORM-d): δ ppm 15.6 (q, 1 C) 16.3 (q, 1 C) 21.5 (q, 1 C) 23.3 (t, 1 C) 24.2 (t, 1 C) 26.4 (q, 1 C) 33.8 (t, 1 C) 35.1 (d, 1 C) 35.4 (t, 1 C) 35.7 (s, 1 C) 38.0 (d, 1 C) 44.7 (t, 1 C) 47.0 (s, 1 C) 50.1 (d, 1 C) 59.8 (t, 1 C) 68.2 (d, 1 C) 95.7 (s, 1 C) 124.1 (d, 1 C) 127.0 (d, 1 C) 140.6 (d, 1 C) 162.5 (s, 1 C) 199.1 (s, 1 C) 210.3 (s, 1 C)
2.5. Biotransformation of 18-methyl-9β,10α-androst-4-ene-3,17-dione with *Amycolatopsis mediterranei* LS30
In an analogous manner to the procedure described in Example 2.1 using 10, 15 or 20 mg 18-methyl-9β,10α-androst-4-ene-3,17-dione as educt, the corresponding 18-methyl-11β-hydroxy-9β,10α-androst-4-ene-3,17-dione was obtained in a yield of 27, 39 and 36%, respectively.
¹³C NMR (126 MHz, CHLOROFORM-d): δ ppm 15.4 (q, 1 C) 22.0 (t, 1 C) 22.3 (q, 1 C) 27.2 (t, 1 C) 29.0 (t, 1 C) 31.1 (d, 1 C) 33.5 (t, 1 C) 35:1 (t, 1 C) 37.8 (t, 1 C) 38.2 (s, 1 C) 38.3 (t, 1 C) 43.0 (d, 1 C) 47.3 (s, 1 C) 55.0 (d, 1 C) 68.6 (d, 1 C) 124.4 (d, 1 C) 170.7 (s, 1 C) 199.2 (s, 1 C) 219.4 (s, 1 C)
2.6. Biotransformation of Dydrogesterone with *Amycolatopsis mediterranei* DSM 43304

In order to show that also other *Amycolatopsis mediterranei* strains are able to transform the retrosteroids of general formula (I) into their corresponding 11-hydroxylated compounds, the fermentation as described in Example 2.1 was repeated with strains available from public culture collections.

*Amycolatopsis mediterranei* DSM 43304 bacterial colonies were transferred from agar plates and grown in 500 ml Erlenmeyer flasks using 100 ml of the nutrient medium. The culture was incubated on a rotary shaker operating at 200 rpm and 30°C. After 2 days of cultivation, 15 mg of. Dydrogesterone was added as a 20 mg/ml solution in DMSO to the culture medium. The resulting solution was fermented at 30°C and 200 rpm for 46 - 50 hours as described hereinabove to promote hydroxylation of the Dydrogesterone. Then, the culture medium was centrifuged (20 min at 17,000 x g). Aliquots of the supernatant were analyzed by HPLC. Then, the supernatant was extracted twice with ¼ volume of ethyl acetate and addition of solid NaCl. The organic phases were combined, dried over NaSO₄, and analyzed by HPLC as set out above. Two independent experiments yielded about 5.7 mg of the desired 11-OH-Dydrogesterone (Yield: 36% without any optimization).

### 3. Isolation of the 11β-OH Dydrogesterone from the bacterial culture medium

Three individual fermentation reactions of Dydrogesterone in 22 1, 8.8 I and 8.8 I were performed as described in Example 2.2. Taken together, 5.88 g of Dydrogesterone were added to the nutrient medium. After termination of the fermentation reaction (after about 47 hours), the medium containing the desired 11β-hydroxy Dydrogesterone was immediately purified by microfiltration (pore size of the membrane 0.2 - 0.3 µm). By HPLC analysis, the yield of 11β-OH Dydrogesterone in the three fermentation batches was determined to be from about 28 to 64%, whereby the lowest value was from a fermentation batch with exceptional low performance. The obtained filtrate (= supernatant) of the three batches was combined to yield 37.9 I of supernatant material containing about 0.07 mg/ml 11β-OH Dydrogesterone.
3.1. Adsorption of the steroid content of the fermentation supernatant on a semi-polar polyacrylic ester adsorber resin
A column having a height of 27 cm and a diameter of 6.5 cm was filled with 1000 ml of a semi-polar polyacrylic ester adsorber resin (=Amberlite XAD-7 HP^{™}, manufactured by Rohm and Haas) swollen in water. The column was equilibrated by 4 bed volumes (BV) of 99% Ethanol and 5 BV of water. 37.9 liters of fermentation supernatant (for dry matter content (=DM) and also contents of 11-Hydroxy-dydrogesterone, 9-Hydroxy-dydrogesterone, and Dydrogesterone were determined by means of HPLC) were passed through the column at room temperature at a flow rate of about 110 ml/min (=approximately 6.5 BV per hour). The steroid content of the fermentation solution was fully adsorbed on the semi-polar adsorber resin column. The steroid content of the urine effluent was determined by HPLC, and the effluent proved to be practically steroid-free. The bottom product was discarded.
3.2. Alkaline washing of the laden adsorber resin column
The steroid-charged adsorber resin column was washed with 5 I of an aqueous 2% sodium hydroxide solution having a pH value of approximately 14. To this end, the alkaline washing water was passed through the column at a flow rate of 90 - 95 ml/min. (=approximately 5.5 - 5.7 BV per hour).
The contents of the different retrosteroids in the washing liquid effluent were analyzed by HPLC. The analysis showed that less than 1% of the total steroids charged onto the column were washed out during the washing phase.
3.3. Intermediate washing of the laden adsorber resin column
The washed and steroid-charged adsorber resin column was washed with 5 I of water. To this end, the water was passed through the column at a flow rate of 95 ml/min. (=approximately 5.7 BV per hour). The contents of the different retrosteroids in the washing liquid effluent were analyzed by HPLC. The analysis showed that nearly none of the total steroids charged onto the column was washed out during the intermediate washing phase.
3.4. Desorption of the conjugated estrogens from the washed adsorber resin column
6 I of the elution liquid (99% Ethanol) were passed through the column at room temperature at a flow rate of approximately 95 ml/min. The eluate running off was collected in 6 fractions. Each fraction was about 1000 ml (=approximately 1 BV). The contents of the individual retrosteroids in the fractions were analyzed by HPLC. Approximately 92% of the total quantity of 11β-OH Dydrogesterone adsorbed on the column was contained in the fractions 2 and 3. Optionally, the remaining fractions can be returned to method step b) after the solvent content has been distilled off.
The dry matter content in % by weight and the respective contents of 11-Hydroxy-dydrogesterone, 9-Hydroxy-dydrogesterone, and Dydrogesterone as determined by HPLC are given in the following table 3 (on the next page).
3.5. Regeneration of the adsorber resin column
In order to regenerate the column, it was washed with 5 I (= 5 BV) of water. The column can be charged and regenerated many times, for instance up to 40 times.

**Table 3: Analysis of the adsorber chromatography**

| | **Vol** | **M** | **DM** | **Dydro** | **9-OH Dydro** | **11-OH Dydro** | **11-OH Dydro** | **11-OH Dydro** |
|---|---|---|---|---|---|---|---|---|
| | **[I]** | **[g]** | **weight %** | **[mg/l]** | **[mg/l]** | **[mg/l]** | **[mg]** | **Yield** % |
| **Fermenter solution** | 37.9 | ~37.9 | 6.1 | 0.002 | n.d. | 0.07 | 2,653 | 43.99 |
| **Alkaline Washing** | | | | | | | | |
| **A1** | 1.0 | | n.d. | 0.0 | 0.0 | 0.016 | 15.7 | |
| **A2** | 1.0 | | n.d. | 0.0 | 0.0 | | | |
| **A3** | 1.0 | | n.d. | 0.0 | 0.0 | | | |
| **A4** | 1.0 | | n.d. | 0.0 | 0.0 | | | |
| **A5** | 1.0 | | n.d. | 0.0 | 0.0 | | | |
| **Intermediate Washing** | | | | | | | | |
| **I1** | 1.0 | | n.d. | 0.0 | 0.0 | 0.0 | 0.0 | 0.0 |
| **I2** | 1.0 | | n.d. | 0.0 | 0.0 | 0.001 | 1.0 | 0.02 |
| **I3** | 1.0 | | n.d. | 0.0 | 0.0 | 0.001 | 0.7 | 0.01 |
| **I4** | 1.0 | | n.d. | 0.0 | 0.0 | 0.001 | 0.7 | 0.01 |
| **I5** | 1:0 | | n.d. | 0.0 | 0.0 | 0.001 | 0.6 | 0.01 |
| **Elution** | | | | | | | | 0.00 |
| **E1** | 1.0 | 970.95 | 0.25 | 0.003 | 0.040 | 0.095 | 95.5 | 1.58 |
| **E2** | 1.0 | 813.82 | 0.80 | 0.101 | 0.636 | 2.007 | 2,007.4 | 33.29 |
| **E3** | 1.0 | 764.80 | 0.05 | 0.032 | 0.103 | 0.269 | 268.7 | 4.46 |
| **E4** | 1.0 | 765.35 | 0.02 | 0.010 | 0.029 | 0.059 | 58.9 | 0.98 |
| **E5** | 1.0 | | 0.01 | 0.003 | 0.009 | 0.012 | 12.4 | 0.21 |
| **E6** | 1.0 | | 0.01 | 0.001 | 0.001 | 0.003 | 2.6 | 0.04 |
| **E1-4^{**}** | **4.0** | **3314.92** | **0.28** | **0.036** | **0.202** | **0.608** | **2,430.5** | **40.30** |

| | | | | | | | | |
|---|---|---|---|---|---|---|---|---|
| * in comparison to the starting amount of 5.88 g Dydrogesterone (= 18.8 mmol) ** calculated | | | | | | | | |

3.6. Further Work-up of the eluate fractions
The eluate fractions 1 to 4 (E1 = 970.95 g, E2 = 813.82 g, E3 = 764.80 g, E4 = 765.35 g) were mixed and reduced from 3314.92 g (DM = 0.26%) to 355.12 g by vaporization at 60°C. The steroid precipitated from the obtained suspension (DM of the supernatant = 2.0%) and was isolated by suction filtration (Yield: about 2.2 g of dried precipitate). From the remaining supernatant a second precipitate could be obtained by further volume reduction down to 50.0 g by vaporization.
The combined precipitates (about 4 g) were further purified by flash chromatography (eluent: ethylacetate:cyclohexane 2:1 changing to pure ethylacetate). The impurity contained in the original ' precipitate could be identified as 9-Hydroxy-dydrogesterone. Finally, 2.15 g of 11-Hydroxy-dydrogesterone were obtained (overall yield: 35%).
¹³C NMR (101 MHz, CHLOROFORM-d): δ ppm 14.7 (q, 1 C) 21.7 (q, 1 C) 22.6 (t, 1 C) 24.8 (t, 1 C) 31.2 (q, 1 C) 33.7 (t, 1 C) 35.2 (d, 2 C) 35.7 (s, 1 C) 43.4 (s, 1 C) 46.2 (t, 1 C) 49.7 (d, 1 C) 49.9 (d, 1 C) 63.6 (d, 1 C) 67.6 (d, 1 C) 124.2 (d, 1 C) 126.9 (d, 1 C) 140.2 (d, 1 C) 162.2 (s, 1 C) 199.1 (s, 1 C) 208.6 (s, 1C)
¹H NMR (400 MHz, CHLOROFORM-d): δ ppm 0.9 (s, 3 H) 1.2 (s, 3 H) 1.4 - 1.5 (m, 1 H) 1.7 - 2.0 (m, 6 H) 2.2 (s, 3 H) 2.2 - 2.3 (m, 1 H) 2.3 - 2.4 (m, 2 H) 2.4 - 2.6 (m, 3 H) 2.7 (ddd, J=12.0, 5.8, 5.6 Hz, 1 H) 4.4 - 4.5 (m, 1 H) 5.7 - 5.7 (m, 1 H) 6.2 - 6.2 (m, 2 H)

The foregoing description and examples have been set forth merely to illustrate the invention and are not intended to be limiting. Since modifications of the described embodiments incorporating the spirit and substance of the invention may occur to persons skilled in the art, the invention should be construed broadly to include all variations falling within the scope of the appended claims and equivalents thereof.

### CITED LITERATURE

- BE 577,615
- BE 652,597
- EP 0 028 309
- EP 0 152 138 B1 (= US 4,601,855)
- EP 0 558 119 B1 (= US 5,304,291)
- GB 1,111,320
- Halkes SJ, Hartog J, Morsink L, de Wachter AM. (1972) "Investigations on sterols. 38. Synthesis of 1,2-methylene-17α-acetoxy-9β,10α-pregnanes, a class of potent progestational agents" Journal of Medicinal Chemistry, 15(12):1288-92
- Halkes SJ & Van Moorselaar R (1969) "Investigations on Sterols XXXIV: Synthesis of 18-methyl-9β,10α-androstanes" Recueil des Travaux Chimiques des Pays-Bas, 1969, 88(7):752-765
- Hartog J, Wittelaar JI, Morsink L, de Wachter AM (1972) "Investigations on sterols. 39. Synthesis and progestational activities of some 16-methylene-17α-acetoxy-9β,10α-pregna-4,6-diene-3,20-dione derivatives" J Med Chem. 1972 Dec;15(12):1292-7
- Lechevalier, M.P., Prauser, H., Labeda, D.P., and Ruan, J.S. (1986) "Two new genera of nocardioform actinomycetes: Amycolata gen. nov. and Amycolatopsis gen. nov." Int. J. Syst. Bacteriol. 36:29-37.
- Margalith, P., and Beretta, G. (1960) "Rifamycin. IX. Taxonomic study on Streptomyces mediterranei sp. nov." Mycopathol. Appl. 13:321-330.
- Saucy G, Els H, Mikesch F, Fürst A (1966) Über 9β,10α-Steroide: Strukturaufklärung von mikrobiologischen Umsetzungsprodukten mit Sauerstoff-Funktion in Stellung 11" Helv Chim Acta 49(5): 1529-1542.
- US 3,304,314
- US 3,555,053
- US 3,937,700
- US 4,353,985
- US 4,588,683
- US 6,046,023
- van der Sijde D, de Flines J, van der Waard WF (1966) "Microbial transformation of 9β,10α-Steroids: III. 11-Hydroxylation and side chain degradation of 9β,10α-Steroids" Recueil des Travaux Chimiques des Pays-Bas, 85:721-730.
- Van Moorselaar R. & Halkes SJ (1969) ."Investigations on Sterols XXXIII : Synthesis of 18-alkyl-9β,10α-pregnane derivatives" Recueil des Travaux Chimiques des Pays-Bas, 88(7):737-51
- Westerhof P. & Hartog J. (1965) "Investigations on Sterols XXIX: Synthesis and properties of some 6,7-dehydro-9β,10α-steroids" Recueil des Travaux Chimiques des Pays-Bas, 84(7):918-31
- Westerhof P., Hartog J. & Halkes SJ (1965) "Investigations on Sterols XXVI: Synthesis and properties of 6-substituted 9β,10α-steroids" Recueil des Travaux Chimiques des Pays-Bas, 84:863-884.
- WO 98/08526
- Zakelj-Mavric M, Plemenitas A, Komel R, Belic I. (1990) "11 beta-hydroxylation of steroids by Cochliobolus lunatus." J Steroid Biochem. 35(5):627-9.

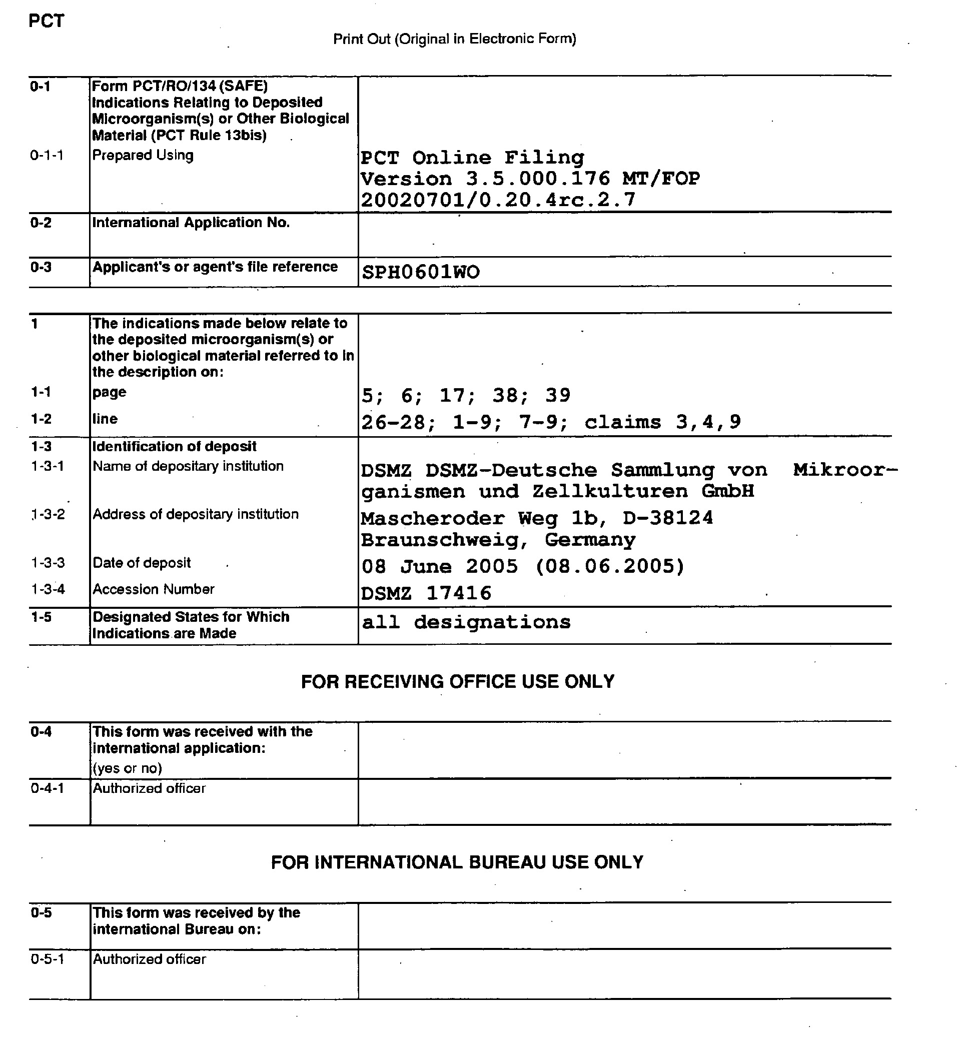

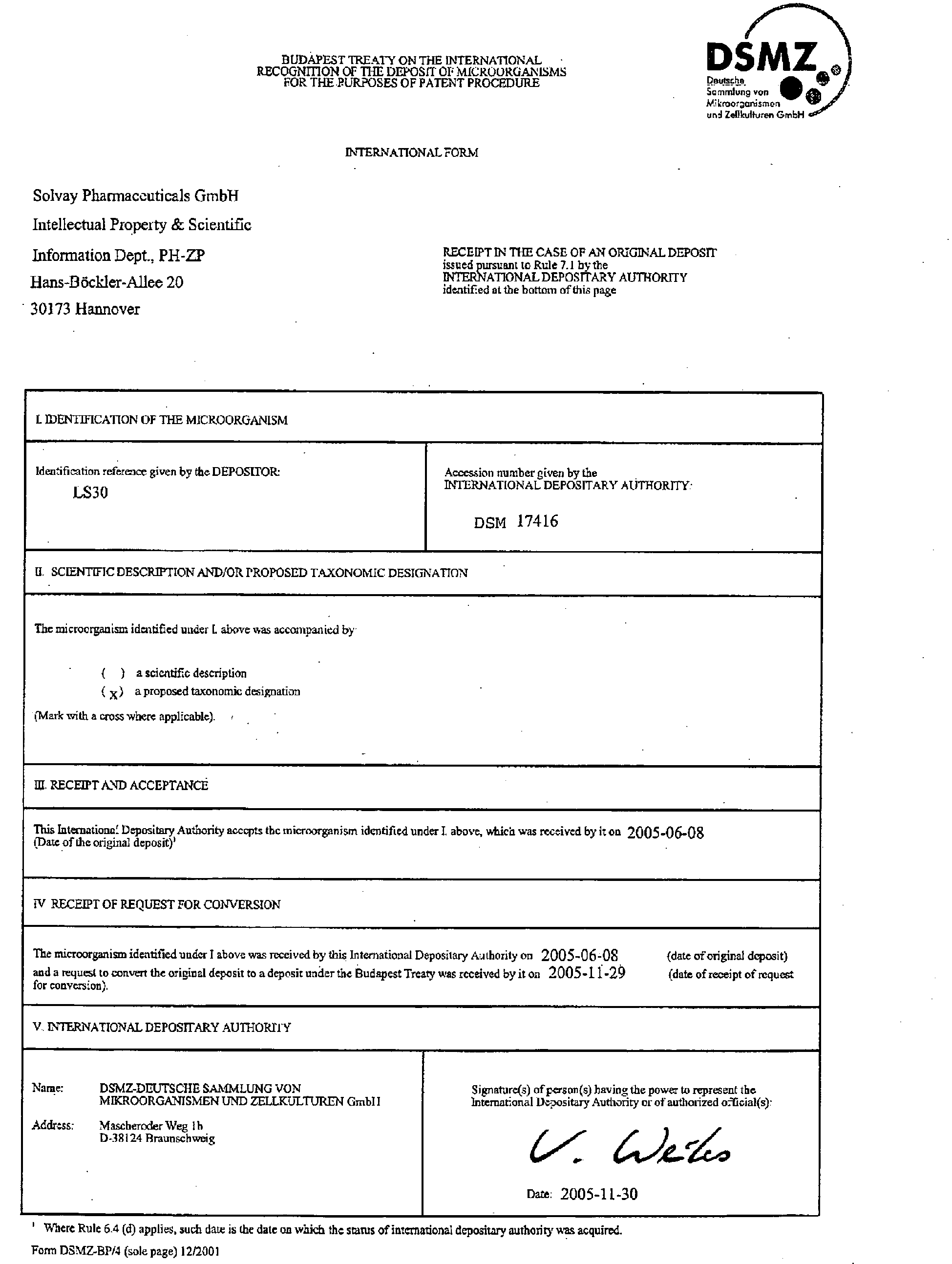

## Claims

1. Use of a bacterial microorganism of the species *Amycolatopsis mediterranei* for the transformation of a 9β,10α-steroidal compound of general Formula (I) 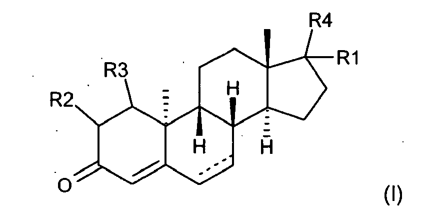 wherein
R1 and R4 together form an oxygen, or
R4 is a β-acetyl group and R1 is selected from the group consisting of hydrogen, -OH, -O-(C₁-C₄)alkyl, and -O-CO-(C₁-C₄)alkyl, and
R2 and R3 are both hydrogen or together form a methylen group,
into its corresponding 11β-hydroxyl analogue.

2. Use according to claim 1, wherein the bacterial microorganism of the species *Amycolatopsis, mediterranei* is a strain selected from the group consisting of LS30, DSM 43304, DSM 40773, and DSM 46096.

3. Use according to claim 2, wherein the strain is *Amycolatopsis mediterranei* LS30 as deposited under DSM 17416.

4. Bacterial strain *Amycolatopsis mediterranei* LS30 as deposited under DSM 17416.

5. A process for the microbial *in vitro* transformation of a 9β,10α-steroidal compound of general Formula (I) 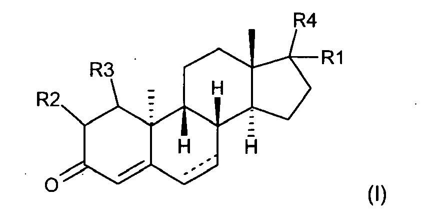 wherein
R1 and R4 together form an oxygen, or
R4 is a β-acetyl group and R1 is selected from the group consisting of hydrogen, -OH, -0-(C₁-C₄)alkyl, and -O-CO-(C₁-C₄)alkyl, and
R2 and R3 are both hydrogen or together form a methylen group;
into its corresponding 11β-hydroxyl analogue, which process comprises contacting a compound of Formula (I) in a fermentation medium with a bacterial member of the species *Amycolatopsis mediterranei* capable of performing the transformation of a compound of Formula (I) into its corresponding 11β-hydroxyl analogue.

6. A process for the microbial *in vitro* transformation according to claim 5, wherein the 9β,10α-steroidal compound is of general Formula (II) 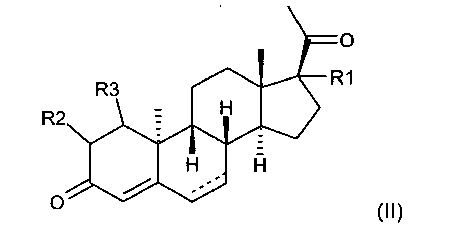 wherein
R1 is selected from the group consisting of hydrogen, -OH, -O-(C₁-C₄)alkyl, and -O-CO-(C₁-C₄)alkyl; and
R2 and R3 are both hydrogen or together form a methylen group.

7. A process for the microbial *in vitro* transformation according to claim 6, wherein the 9β,10α-steroidal compound is of general Formula (III) 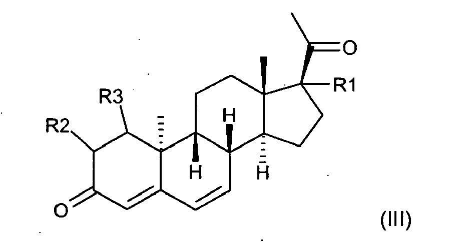 wherein
R1 is hydrogen or -O-(C₁-C₄)alkyl; and
R2 and R3 are both hydrogen or together form a methylen group.

8. A process according to any of the claims 5 to 7, wherein the member of the species *Amycolatopsis mediterranei* is a strain selected from the group consisting of LS30, DSM 43304, DSM 40773, and DSM 46096.

9. A process according to claim 8, wherein the strain is *Amycolatopsis mediterranei* LS30 as deposited under DSM 17416.

10. A process according to any of the claims 5 to 9, wherein at least the transformation process of the fermentation is conducted under aerobic conditions, in particular under a relative O₂ saturation pO₂/pO₂ₘₐₓ of the fermentation medium from about 5% to about 95%, preferably from about 20% to about 80%, more preferably from about 30% to about 75%, and most preferably from about 40% to about 70%.

11. A process according to any of the claims 5 to 10, wherein the *Amycolatopsis mediterranei* bacteria are in the middle or late phase of their exponential growth period when the transformation is started by addition of the 9β, 10α-steroidal compound to the medium.

12. A process according to any of the claims 5 to 11, wherein the 9β,10α-steroidal compound is added in an amount from about 50 mg/l to about 500 mg/l, preferably from about 100 mg/l to about 250 mg/l fermentation medium, and most preferably in an amount of about 150 mg/l fermentation medium.

13. A process according to claim 12, wherein the whole amount of the 9β,10α-steroidal compound is added at once at the beginning of the transformation process or over a period of 1 to 5 hours at the beginning of the transformation process.

14. A process according to claim 12, wherein the amount of the 9β, 10α-steroidal compound is continuously added to the fermentation medium over the complete transformation period.

15. A process according to claim 14, wherein the 9β,10α-steroidal compound is added in a concentration from 1 to 20 mg per hour of cultivation and per liter of fermentation medium, preferably in a concentration from 2 to 5 mg per hour of cultivation and per liter of fermentation medium.

16. A process according to any of the claims 5 to 15, wherein the 11β-hydroxyl analogue is isolated from the fermentation medium by a process comprising the steps of
a) obtaining the supernatant from the fermentation medium optionally freed of any bacterial cells, bacterial debris, mucilaginous substances and solids,
b) contacting a supernatant material selected from the group consisting of the supernatant obtained in step a), a concentrate formed by reducing the volume of said supernatant, and a retentate obtained by membrane filtration of said supernatant, with an amount of a non-ionic semi-polar polymeric adsorber resin sufficient for the adsorption of the 11β-hydroxyl analogue contained in the supernatant material, whereby a non-ionic semi-polar polymeric adsorber resin charged with the 11β-hydroxyl analogue is obtained, and thereafter separating the charged adsorber resin from the rest of the supernatant material;
c) washing the charged adsorber resin with an alkaline aqueous washing liquid having a pH value of at least 12.0, preferably of from 12.5 to 14;
d) optionally performing an intermediate washing step, in which the charged adsorber resin is washed with water; and
e) contacting the washed adsorber resin with an amount of an elution liquid sufficient for the desorption of the 11β-hydroxyl analogue adsorbed thereon, said elution liquid comprising at least one water-miscible organic solvent selected from the group consisting of water-miscible ethers, lower alkanols, and lower aliphatic ketones or a mixture of water which has optionally been rendered alkaline and at least one water-miscible organic solvent selected from the group consisting of water-miscible ethers, lower alkanols and lower aliphatic ketones, and
f) separating an eluate containing the 11β-hydroxyl analogue off from the adsorber resin, and optionally concentrating the eluate by volume reduction.

17. A process according to claim 16, wherein said non-ionic semi-polar polymeric adsorber resin is a macroporous polycarboxylic acid ester resin, preferably a cross-linked aliphatic polycarboxylic acid ester resin, and more preferably a cross-linked polycarboxylic acid ester resin having a macroreticular structure.

18. A process according to claim 16 or 17, wherein in step e) the elution liquid comprises ethanol.

19. An 11β-hydroxy-9β, 10α-steroidal compound of general formula (V) 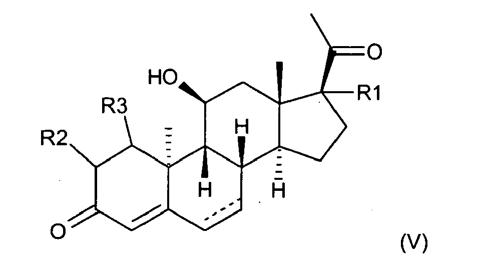 wherein
a) R1 is selected from the group consisting of hydrogen, -OH, -O-(C₁-C₄)alkyl, and -O-CO-(C₁-C₄)alkyl, and
R2 and R3 together form a methylen group, OR
b) R1 is selected from the group consisting of -O-(₁-C₄)alkyl and -O-CO-(C₁-C₄)alkyl, and R2 and R3 both represent hydrogen; OR
c) R1 is -OH,
R2 and R3 both represent hydrogen, and
the compound is a 4,6-diene.

20. An 11β-hydroxy-9β, 10α-steroidal compound according to claim 19, wherein the compound is selected from the group consisting of
11β-Hydroxy-1,2-methylene-9β, 10α-pregna-4,6-diene-3,20-dione,
17α-Ethoxy-11β-hydroxy-9β, 10α-pregna-4,6-diene-3,20-dione,
17α-Ethoxy-11β-hydroxy-1,2-methylene-9β,10 α-pregna-4,6-diene-3,20-dione,
11β-17α-Dihydroxy-9β, 10α-pregna-4,6-diene-3,20-dione,
11β-17α-Dihydroxy-1,2-methylene-9β, 10α-pregna-4,6-diene-3,20-dione,
11β-Hydroxy-1,2-methylene-9β,10α-pregna-4-ene-3,20-dione,
17α-Ethoxy-11β-hydroxy-9β,10α-pregna-4-ene-3,20-dione,
17α-Ethoxy-11β-hydroxy-1,2-methylene-9β,10α-pregna-4-ene-3,20-dione, and
11β-17α-Dihydroxy-1,2-methylene-9β,10α-pregna-4-ene-3,20-dione.

## Patentansprüche

1. Verwendung eines bakteriellen Mikroorganismus der Art *Amycolatopsis mediterranei* zur Umwandlung einer 9β,10α-steroidalen Verbindung der allgemeinen Formel (I) 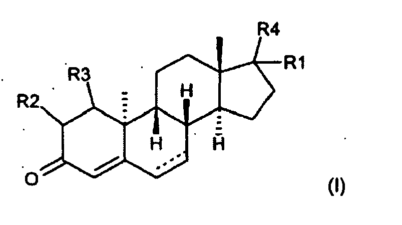 wobei
R1 und R4 zusammen Sauerstoff bilden oder
R4 für eine β-Acetylgruppe steht und R1 aus der aus Wasserstoff, -OH, -O-(C₁-C₄)Alkyl und -O-CO-(C₁-C₄) Alkyl bestehenden Gruppe ausgewählt ist und
R2 und R3 beide für Wasserstoff stehen oder zusammen eine Methylengruppe bilden, in ihr entsprechendes 11β-Hydroxyanalogon.

2. Verwendung nach Anspruch 1, wobei es sich bei dem bakteriellen Mikroorganismus der Art *Amycolatopsis mediterranei* um einen aus der LS30, DSM 43304, DSM 40773 und DSM 46096 bestehenden Gruppe ausgewählten Stamm handelt.

3. Verwendung nach Anspruch 2, wobei es sich bei dem Stamm um als DSM 17416 hinterlegtes *Amycolatopsis mediterranei* LS30 handelt.

4. Bakterienstamm *Amycolatopsis mediterranei* LS30, hinterlegt als DSM 17416.

5. Verfahren zur mikrobiellen in-vitro-Umwandlung einer 9β,10α-steroidalen Verbindung der allgemeinen Formel (I) 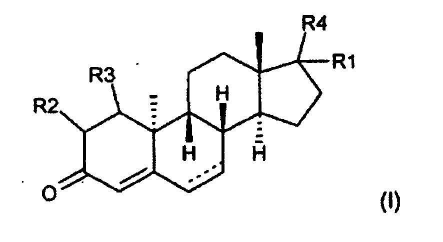 wobei
R1 und R4 zusammen Sauerstoff bilden oder
R4 für eine β-Acetylgruppe steht und R1 aus der aus Wasserstoff, -OH, -O-(C1-C4)Alkyl und -O-CO-(C₁-C₄)Alkyl bestehenden Gruppe ausgewählt ist und
R2 und R3 beide für Wasserstoff stehen oder zusammen eine Methylengruppe bilden,
in ihr entsprechendes 11β-Hydroxyanalogon, wobei man bei dem Verfahren eine Verbindung der Formel (I) in einem Fermentationsmedium mit einem Bakterium der Art *Amycolatopsis mediterranei,* das dazu in der Lage ist, eine Verbindung der Formel (I) in ihr entsprechendes 11β-Hydroxyanalogon umzuwandeln, in Kontakt bringt.

6. Verfahren zur mikrobiellen in-vitro-Umwandlung nach Anspruch 5, wobei die 9β,10α-steroidale Verbindung die allgemeine Formel (II) 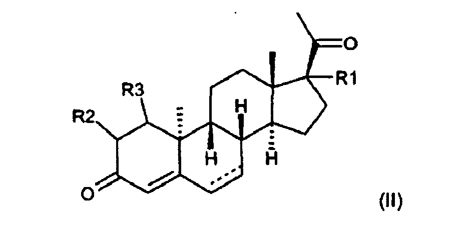 aufweist, wobei
R1 aus der aus Wasserstoff -OH, -O-(C₁-C₄)Alkyl und -O-CO-(C₁-C₄)Alkyl bestehenden Gruppe ausgewählt ist und
R2 und R3 beide für Wasserstoff stehen oder zusammen eine Methylengruppe bilden.

7. Verfahren zur mikrobiellen in-vitro-Umwandlung nach Anspruch 6, wobei die 9β,10α-steroidale Verbindung die allgemeine Formel (III) 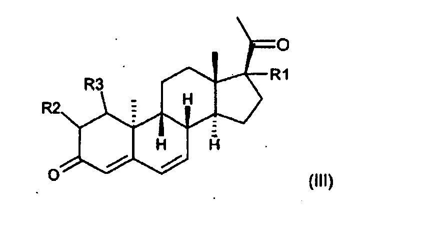 aufweist, wobei
R1 für Wasserstoff oder -O-(C₁-C₄)Alkyl steht und
R2 und R3 beide für Wasserstoff stehen oder zusammen eine Methylengruppe bilden.

8. Verfahren nach einem der Ansprüche 5 bis 7, wobei es sich bei dem Bakterium der Art *Amycolatopsis mediterranei* um einen aus der LS30, DSM 43304, DSM 40773 und DSM 46096 bestehenden Gruppe ausgewählten Stamm handelt.

9. Verfahren nach Anspruch 8, wobei es sich bei dem Stamm um als DSM 17416 hinterlegtes *Amycolatopsis mediterranei* LS30 handelt.

10. Verfahren nach einem der Ansprüche 5 bis 9, wobei wenigstens das Umwandlungsverfahren der Fermentation unter aeroben Bedingungen, insbesondere unter einer relativen O₂-Sättigung pO₂/pO₂ₘₐₓ des Fermentationsmediums von etwa 5% bis etwa 95%, vorzugsweise von etwa 20% bis etwa 80%, besonders bevorzugt von etwa 30% bis etwa 75% und ganz besonders bevorzugt von etwa 40% bis etwa 70% durchgeführt wird.

11. Verfahren nach einem der Ansprüche 5 bis 10, wobei sich die *Amycolatopsis-mediterranei-Bakterien* in der mittleren oder späten Phase ihrer exponentiellen Wachstumsperiode befinden, wenn die Umwandlung durch Zugabe der 9β,10α-steroidalen Verbindung zum Medium gestartet wird.

12. Verfahren nach einem der Ansprüche 5 bis 11, wobei die 9β,10α-steroidale Verbindung in einer Menge von etwa 50 mg/l bis etwa 500 mg/l, vorzugsweise von etwa 100 mg/l bis etwa 250 mg/l Fermentationsmedium und ganz besonders bevorzugt in einer Menge von etwa 150 mg/l Fermentationsmedium zugesetzt wird.

13. Verfahren nach Anspruch 12, wobei die Gesamtmenge der 9β,10α-steroidalen Verbindung in einer Portion zu Beginn des Umwandlungsverfahrens oder über einen Zeitraum von 1 bis 5 Stunden zu Beginn des Umwandlungsverfahrens zugesetzt wird.

14. Verfahren nach Anspruch 12, wobei die Menge der 9β,10α-steroidalen Verbindung dem Fermentationsmedium über die gesamte Umwandlungsperiode kontinuierlich zugesetzt wird.

15. Verfahren nach Anspruch 14, wobei die 9β,10α-steroidale Verbindung in einer Konzentration von 1 bis 20 mg pro Kultivierungsstunde und pro Liter Fermentationsmedium, vorzugsweise in einer Konzentration von 2 bis 5 mg pro Kultivierungsstunde und pro Liter Fermentationsmedium, zugesetzt wird.

16. Verfahren nach einem der Ansprüche 5 bis 15, wobei das 11β-Hydroxyanalogon aus dem Fermentationsmedium durch ein Verfahren isoliert wird, bei dem man
a) den Überstand vom Fermentationsmedium gewinnt, gegebenenfalls frei von Bakterienzellen, Bakterientrümmern, Schleimsubstanzen und Feststoffen,
b) ein Überstandmaterial ausgewählt aus der Gruppe bestehend aus dem im Schritt a) erhaltenen Überstand, einem durch Einengen des Volumens dieses Überstands gebildeten Konzentrat und einem durch Membranfiltration dieses Überstands erhaltenen Retentats, mit einer für die Adsorption des im Überstandmaterial enthaltenen 11β-Hydroxyanalogons ausreichenden Menge eines nichtionischen semipolaren polymeren Adsorberharzes in Kontakt bringt, wodurch man ein mit dem 11β-Hydroxyanalogon beladenes nichtionisches semipolares polymeres Adsorberharz erhält, und anschließend das beladene Adsorberharz vom Rest des Überstandmaterials abtrennt;
c) das beladene Adsorberharz mit einer alkalischen wäßrigen Waschflüssigkeit mit einem PH-Wert von mindestens 12,0, vorzugweise von 12,5 bis 14, wäscht;
d) gegebenenfalls einen Wasch-Zwischenschritt durchführt, bei dem das beladene Adsorberharz mit Wasser gewaschen wird;
e) das gewaschene Adsorberharz mit einer zur Desorption des daran adsorbierten 11β-Hydroxyanalogons ausreichenden Menge einer Elutionsflüssigkeit in Kontakt bringt, wobei die Elutionsflüssigkeit wenigstens ein mit Wasser mischbares organisches Lösungsmittel ausgewählt aus der Gruppe bestehend aus mit Wasser mischbaren Ethern, niederen Alkanolen und niederen aliphatischen Ketonen oder eine Mischung von Wasser, das gegebenenfalls alkalisch gestellt wurde, und wenigstens einem mit Wasser mischbaren organischen Lösungsmittel ausgewählt aus der Gruppe bestehend aus mit Wasser mischbaren Ethern, niederen Alkanolen und niederen aliphatischen Ketonen umfaßt, und
f) ein das 11β-Hydroxyanalogon enthaltende Eluat vom Adsorberharz abtrennt, und gegebenenfalls das Eluat durch Volumenreduktion einengt.

17. Verfahren nach Anspruch 16, wobei es sich bei dem nichtionischen semipolaren polymeren Adsorberharz um ein makroporöses Polycarbonsäureesterharz, vorzugsweise ein quervernetztes aliphatisches Polycarbonsäureesterharz und besonders bevorzugt ein quervernetztes Polycarbonsäureesterharz mit einer makroretikulären Struktur handelt.

18. Verfahren nach Anspruch 16 oder 17, wobei in Schritt e) die Elutionsflüssigkeit Ethanol enthält.

19. 11β-Hydroxy-9β,10α-steroidale Verbindung der allgemeinen Formel (V) 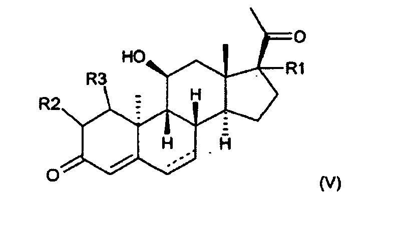 wobei
a) R1 aus der aus Wasserstoff, -OH, -O-(C₁-C₄)Alkyl und -O-CO- (C₁-C₄) Alkyl bestehenden Gruppe ausgewählt ist und
R2 und R3 zusammen eine Methylengruppe bilden, oder
b) R1 aus der aus -O- (C1-C4)Alkyl und -O-CO-(C₁-C₄)Alkyl bestehenden Gruppe ausgewählt ist und R2 und R3 beide für Wasserstoff stehen, oder
c) R1 für -OH steht,
R2 und R3 beide für Wasserstoff stehen und
es sich bei der Verbindung um ein 4,6-Dien handelt.

20. 11β-Hydroxy-9β,10α-steroidale Verbindung nach Anspruch 19, wobei die Verbindung ausgewählt ist aus der Gruppe bestehend aus
11β-Hydroxy-1,2-methylen-9β,10α-pregna-4,6-dien-3, 20-dion,
17α-Ethoxy-11β-hydroxy-9β,10α-pregna-4,6-dien-3,20 -dion,
17α-Ethoxy-11β-hydroxy-1,2-methylen-9β,10α-pregna-4,6-dien-3,20-dion,
11β-17α-Dihydroxy-9β,10α-pregna-4,6-dien-3,20-dion,
11β-17α-Dihydroxy-1,2-methylen-9β,10α-pregna-4,6-dien-3,20-dion,
11β-Hydroxy-1,2-methylen-9β,10α-pregna-4-en-3,20-dion,
17α-Ethoxy-11β-hydroxy-9β,10α-pregna-4-en-3,20-dion,
17α-Ethoxy-11β-hydroxy-1,2-methylen-9β,10α-pregna-4-en-3,20-dion, und
11β-17α-Dihydroxy-1,2-methylen-9β,10α-pregna-4-en-3,20-dion.

## Revendications

1. Utilisation d'un microorganisme bactérien de l'espèce *Amycolatopsis mediterranei* pour la transformation d'un composé 9β,10α-stéroïdien de formule générale (I) 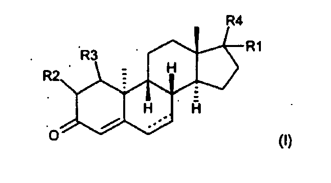 dans laquelle
R1 et R4 forment ensemble un atome d'oxygène, ou
R4 est un groupe β-acétyle et R1 est choisi dans le groupe constitué d'un atome d'hydrogène, -OH, - O-(C₁-C₄)alkyle et -O-CO- (C₁-C₄) alkyle, et
R2 et R3 sont tous deux un atome d'hydrogène ou forment ensemble un groupe méthylène,
en son analogue 11β-hydroxyle correspondant.

2. Utilisation selon la revendication 1, selon laquelle le microorganisme bactérien de l'espèce *Amycolatopsis mediterranei* est une souche choisie dans le groupe constitué de LS30, DSM 43304, DSM 40773 et DSM 46096.

3. Utilisation selon la revendication 2, selon laquelle la souche est *Amycolatopsis mediterranei* LS30 telle que déposée sous le numéro DSM 17416.

4. Souche bactérienne *Amycolatopsis mediterranei* LS30 telle que déposée sous le numéro DSM 17416.

5. Procédé de transformation microbienne *in vitro* d'un composé 9β,10α-stéroïdien de formule générale (I) 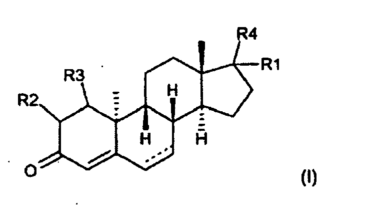 dans laquelle
R1 et R4 forment ensemble un atome d'oxygène, ou
R4 est un groupe β-acétyle et R1 est choisi dans le groupe constitué d'un atome d'hydrogène, -OH, - O-(C₁-C₄) alkyle et -O-CO-(C₁-C₄)alkyle, et
R2 et R3 sont tous deux un atome d'hydrogène ou forment ensemble un groupe méthylène ;
en son analogue 11β-hydroxyle correspondant, ledit procédé comprenant la mise en contact d'un composé de formule (I) dans un milieu de fermentation avec un membre bactérien de l'espèce *Amycolatopsis mediterranei* capable d'effectuer la transformation d'un composé de formule (I) en son analogue 11β-hydroxyle correspondant.

6. Procédé de transformation microbienne *in vitro* selon la revendication 5, selon lequel le composé 9β,10α-stéroidien est de formule générale (II) 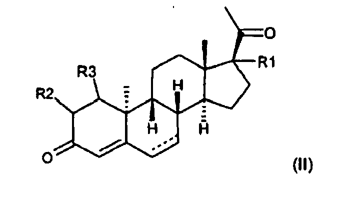 dans laquelle
R1 est choisi dans le groupe constitué d'un atome d'hydrogène, -OH, -O-(C₁-C₄)alkyle et -O-CO- (C₁-C₄)alkyle, et
R2 et R3 sont tous deux un atome d'hydrogène ou forment ensemble un groupe méthylène.

7. Procédé de transformation microbienne *in vitro* selon la revendication 6, selon lequel le composé 9β, 10α-stéroïdien est de formule générale (III) 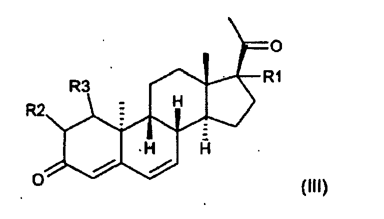 dans laquelle
R1 est un atome d'hydrogène ou -O-(C₁-C₄)alkyle, et R2 et R3 sont tous deux un atome d'hydrogène ou forment ensemble un groupe méthylène.

8. Procédé selon l'une quelconque des revendications 5 à 7, selon lequel le membre de l'espèce *Amycolatopsis mediterranei* est une souche choisie dans le groupe constitué de LS30, DSM 43304, DSM 40773 et DSM 46096.

9. Procédé selon la revendication 8, selon lequel la souche est *Amycolatopsis mediterranei* LS30 telle que déposée sous le numéro DSM 17416.

10. Procédé selon l'une quelconque des revendications 5 à 9, selon lequel au moins le procédé de transformation de la fermentation est mené dans des conditions aérobies, en particulier à une saturation en O₂ relative pO₂/pO₂ₘₐₓ du milieu de fermentation comprise entre environ 5 % et environ 75 %, et plus préférablement comprise entre environ 40 % et environ 70 %.

11. Procédé selon l'une quelconque des revendications 5 à 10, selon lequel les bactéries *Amycolatopsis mediterranei* se trouvent dans la phase moyenne ou tardive de leur période de croissance exponentielle lorsque la transformation est lancée par l'ajout du composé 9β,10α-stéroïdien dans le milieu.

12. Procédé selon l'une quelconque des revendications 5 à 11, selon lequel le composé 9β,10α-stéroïdien est ajouté en une quantité comprise entre environ 50 mg/l et environ 500 mg/l, de préférence comprise entre environ 100 mg/l et environ 250 mg/l de milieu de fermentation, et le plus préférablement en une quantité environ égale à 150 mg/l de milieu de fermentation.

13. Procédé selon la revendication 12, selon lequel la quantité totale du composé 9β,10α-stéroidien est ajoutée en une seule fois au début du procédé de transformation ou sur une période de 1 à 5 heures au début du procédé de transformation.

14. Procédé selon la revendication 12, selon lequel la quantité de composé 9β,10α-stéroïdien est ajoutée en continu dans le milieu de fermentation sur l'ensemble de la période de transformation.

15. Procédé selon la revendication 14, selon lequel le composé 9β,10α-stéroidien est ajouté en une concentration comprise entre 1 et 20 mg par heure de mise en culture et par litre de milieu de fermentation, de préférence en une concentration comprise entre 2 et 5 mg par heure de mise en culture et par litre de milieu de fermentation.

16. Procédé selon l'une quelconque des revendications 5 à 15, selon lequel l'analogue 11β-hydroxyle est isolé à partir du milieu de fermentation par un procédé comprenant les étapes consistant à :
a) obtenir le surnageant à partir du milieu de fermentation éventuellement débarrassé de toute cellule bactérienne, de tout débris cellulaire, de toute substance mucilagineuse et de tout solide,
b) mettre en contact un matériel de surnageant choisi dans le groupe constitué du surnageant obtenu à l'étape a), d'un concentré formé en réduisant le volume dudit surnageant et d'un rétentat obtenu par filtration sur membrane dudit surnageant, avec une quantité de résine d'adsorption polymère semi-polaire non ionique suffisante pour permettre l'adsorption de l'analogue 11β-hydroxyle contenu dans le matériel de surnageant, ce par quoi une résine d'adsorption polymère semi-polaire non ionique chargée de l'analogue 11β-hydroxyle est obtenue, et par la suite séparer la résine d'adsorption chargée du reste du matériel de surnageant ;
c) laver la résine d'adsorption chargée avec un liquide de lavage aqueux alcalin ayant une valeur de pH au moins égale à 12,0, de préférence comprise entre 12,5 et 14 ;
d) réaliser éventuellement une étape de lavage intermédiaire dans laquelle la résine d'adsorption chargée est lavée avec de l'eau ; et
e) mettre en contact la résine d'adsorption lavée avec une quantité d'un liquide d'élution suffisante pour permettre la désorption de l'analogue 11β-hydroxyle adsorbé sur celle-ci, ledit liquide d'élution comprenant au moins un solvant organique miscible à l'eau choisi dans le groupe constitué d'éthers miscibles à l'eau, d'alcanols inférieurs et de cétones aliphatiques inférieures ou un mélange d'eau qui a éventuellement été rendue alcaline et d'au moins un solvant organique miscible à l'eau choisi dans le groupe constitué d'éthers miscibles à l'eau, d'alcanols inférieurs et de cétones aliphatiques inférieures, et
f) séparer un éluat contenant l'analogue 11β-hydroxyle de la résine d'adsorption et éventuellement concentrer l'éluat par réduction du volume.

17. Procédé selon la revendication 16, selon lequel ladite résine d'adsorption polymère semi-polaire non ionique est une résine d'ester d'acide polycarboxylique macroporeuse, de préférence une résine d'ester d'acide polycarboxylique aliphatique réticulée, et plus préférablement une résine d'ester d'acide polycarboxylique réticulée présentant une structure macroréticulaire.

18. Procédé selon la revendication 16 ou 17, selon lequel dans l'étape e), le liquide d'élution comprend de l'éthanol.

19. Composé 11β-hydroxy-9β,10α-stéroïdien de formule générale (V) 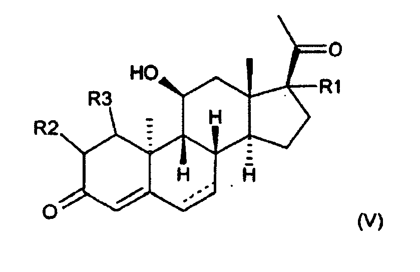 dans laquelle
a) R1 est choisi dans le groupe constitué d'un atome d'hydrogène, -OH, -O-(C₁-C₄)alkyle et -O-CO-(C₁-C₄) alkyle, et
R2 et R3 forment ensemble un groupe méthylène, OU
b) R1 est choisi dans le groupe constitué de -O-(C₁-C₄) alkyle et -O-CO-(C₁-C₄) alkyle, et
R2 et R3 représentent tous deux un atome d'hydrogène, OU
c) R1 est -OH,
R2 et R3 représentent tous deux un atome d'hydrogène, et
le composé est un 4,6-diène.

20. Composé 11β-hydroxy-9β,10α-stéroïdien selon la revendication 19, le composé étant choisi dans le groupe constitué de :
la 11β-hydroxy-1,2-méthylène-9β,10α-prégna-4,6-diène-3,20-dione,
la 17α-éthoxy-11β-hydroxy-9β,10α-prégna-4,6-diène-3,20-dione,
la 17α-éthoxy-11β-hydroxy-1,2-méthylène-9β,10α-prégna-4,6-diène-3,20-dione,
la 11β-17α-dihydroxy-9β,10α-prégna-4,6-diène-3,20-dione,
la 11β-17α-dihydroxy-1,2-méthylène-9β,10α-prégna-4,6-diène-3,20-dione,
la 11β-hydroxy-1,2-méthylène-9β,10α-prégna-4-ène-3,20-dione,
la 17α-éthoxy-11β-hydroxy-9β,10α-prégna-4-ène-3,20-dione,
la 17α-éthoxy-11β-hydroxy-1,2-méthylène-9β,10α-prégna-4-ène-3,20-dione et
la 11β-17α-dihydroxy-1,2-méthylène-9β,10α-prégna-4-ène-3,20-dione.
